(19) Europäisches Patentamt
European Patent Office
Office européen des brevets

(11) **EP 1 800 626 A2**

(12) **EUROPEAN PATENT APPLICATION**

(43) Date of publication:
**27.06.2007 Bulletin 2007/26**

(51) Int Cl.:
***A61F 2/34*** *(2006.01)*     ***A61B 17/86*** *(2006.01)*

(21) Application number: **06256412.5**

(22) Date of filing: **16.12.2006**

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br>Designated Extension States:<br>**AL BA HR MK RS YU** | (72) Inventors:<br>• **Lewis, Paul Peter Philip**<br>  **Warsaw, IN 46580 (US)**<br>• **Christie, Michael J.**<br>  **Nashville, IN 37220 (US)**<br>• **Hugate, Ronald R., Jr.**<br>  **Rochester, MN 55901 (US)** |
| (30) Priority: **21.12.2005 US 314490** | |
| (71) Applicant: **DePuy Products, Inc.**<br>**Warsaw, IN 46581 (US)** | (74) Representative: **Belcher, Simon James**<br>**Urquhart-Dykes & Lord LLP**<br>**Tower North Central**<br>**Merrion Way**<br>**Leeds LS2 8PA (GB)** |

(54) **Modular hip cup assembly**

(57) An acetabular cup assembly (200) for attachment to a acetabulum comprises an acetabular cup (202) having a first connector. A fastener (210) cooperates with the cup and has a second connector. The first and second connectors cooperate to connect the fastener to the cup. The fastener comprises a fastening portion for cooperation with the acetabulum and a connecting portion for connection with the cup. The fastener can be used with a bushing (366) which has a generally spherical outer periphery and an interior wall defining central opening extending through it, the interior wall cooperating with the connecting portion of the fastener.

Fig. 1

EP 1 800 626 A2

**Description**

The present invention relates generally to the field of orthopaedics, and more particularly, to a device for securing a prosthetic component to bone for use in orthopaedic trauma or orthopaedic joint products.

The skeletal system includes many long bones that extend from the human torso. These long bones include the femur, fibula, tibia, humerus, radius and ulna.

A joint within the human body forms a juncture between two or more bones or other skeletal parts. The ankle, hip, knee, shoulder, elbow and wrist are just a few examples of the multitude of joints found within the body. As should be apparent from the above list of examples of joints, many of the joints permit relative motion between the bones. For example, the motion of sliding, gliding, hinge or ball and socket movements may be had by a joint. For example, the ankle permits a hinge movement, the knee allows for a combination of gliding and hinge movements and the shoulder and hip permit movement through a ball and socket arrangement.

The joints in the body are stressed or can be damaged in a variety of ways. For example, gradual wear and tear is imposed on the joints through the continuous use of a joint over the years. The joints that permit motion have cartilage positioned between the bones providing lubrication to the motion and also absorbing some of the forces direct to the joint. Over time, the normal use of a joint may wear down the cartilage and bring the moving bones in direct contact with each other. In contrast, in normal use, a trauma to a joint, such as the delivery of a large force, from an accident for, example, an automobile accident, may cause considerable damage to the bones, the cartilage or to other connective tissue such as tendons or ligaments.

Arthropathy, a term referring to a disease of the joint, is another way in which a joint may become damaged. Perhaps the best known joint disease is arthritis, which is generally referred to as a disease or inflammation of a joint that results in pain, swelling, stiffness, instability, and often deformity.

There are many different forms of arthritis, with osteoarthritis being the most common and resulting from the wear and tear of cartilage within a joint. Another type of arthritis is osteonecrosis, which is caused by the death of a part of the bone due to loss of blood supply. Other types of joint disease are caused by trauma to the joint while others, such as rheumatoid arthritis, Lupus, and psoriatic arthritis destroy cartilage and are associated with the inflammation of the joint lining.

The hip joint is one that is commonly afflicted with arthropathy. The hip joint is a ball and socket joint that joins the femur or thighbone with the pelvis. The pelvis has a hemispherical socket called the acetabulum for receiving a ball socket head in the femur. Both the head of the femur and the acetabulum are coated with cartilage for allowing the femur to move easily within the pelvis. Other joints commonly afflicted with arthropathy include the spine, knee, shoulder, carpals, metacarpals, and phalanges of the hand. Arthroplasty as opposed to arthropathy commonly refers to the making of an artificial joint. In severe cases of arthritis or other forms of arthropathy, such as when pain is overwhelming or when a joint has a limited range of mobility, a partial or total replacement of the joint within an artificial joint may be justified. The procedure for replacing the joint varies, of course, with the particular joint in question, but in general involves replacing a terminal portion of an afflicted bone with a prosthetic implant and inserting a member to serve as a substitute for the cartilage.

The prosthetic implant is formed of a rigid material that becomes bonded with the bone and provides strength and rigidity to the joint and the cartilage substitute members chosen to provide lubrication to the joint and to absorb some of the compressive forces. Suitable material for the implant include metals, and composite materials such as titanium, cobalt chromium, stainless steel, ceramic and suitable materials for cartilage substitutes include polyethylene. A cement may also be used to secure the prosthetic implant to the host bone.

A total hip replacement, for example, involves removing the ball shaped head of the femur and inserting a stem implant into the centre of the bone, which is referred to as the medullary canal, or marrow of the bone. The stem implant may be cemented into the medullary canal or may have a porous coated surface for allowing the bone to heal directly to the implant. The stem implant has a neck and a ball shaped head, which are intended to perform the same functions as a healthy femur's neck and a ball shaped head. The polyethylene cup is inserted into the acetabulum and has a socket for receiving the head on the stem implant.

The polyethylene cup may be positioned directly into the acetabulum. Preferably, the polyethylene cup is secured to a metal member which is in turn secured to the acetabulum. This metal member is typically called a cup or a shell. The cup or shell may include a porous coating for promoting bony in-growth to secure the shell to the acetabulum. Alternatively or in addition the shell may include an opening or a plurality of openings for receiving bone screws to assist in the attachment of the shell to the acetabulum. As an alternative to the polyethylene cup, a cup of a different material may be inserted into the shell. For example, the cup may be made of a metal, for example, cobalt chromium, stainless steel, or titanium. Alternatively, the cup may be made of a ceramic.

Orthopaedic joint reconstruction implants require fixation to bone. For many orthopaedic implant components, the fixation to bone occurs by a stem in the implant component fitted to the medullary canal of the bone. Such fixation is generally fairly effective. However, the fixation of the acetabulum component of the orthopaedic hip joint does not have the benefit of fitting to a medullary canal. The natural acetabulum is reamed into a concave spherical shape with a grater type reamer and the convex spherical outer periphery of the acetabulum component is placed into the reamed acetabulum. A porous coating may be placed on the convex outer periphery of the cup or shell for promoting bony ingrowth with the reamed acetabulum. Alternatively, the cup may include an external thread or groove on its periphery, which can be threadably secured to the acetabulum. Further, the cup may be secured to the acetabulum by means of screws positioned through openings in the cup.

The present methods of securing the cup to the acetabulum work fairly well in patients with good pelvic bone. However, for those patients in which the pelvic bone is of poor quality or includes significant defects, the fixation of the hip cup to the acetabulum may not be secure.

Current available options include the use of non-locking screws, which can allow the acetabular cup to migrate or change position relative to the screws that are used to attach the acetabular cup to the pelvis. Non-locking screws effectively sandwich the acetabular device or cup between the screw head and the bone in which the screw is anchored. If the bone screw interface is disrupted, or if the bone resorbs over time behind the acetabular cup, the sandwich affect of the cup position between the head of the screw and the threads of the screw loosens its grip on the acetabular cup and allows the acetabular cup to move.

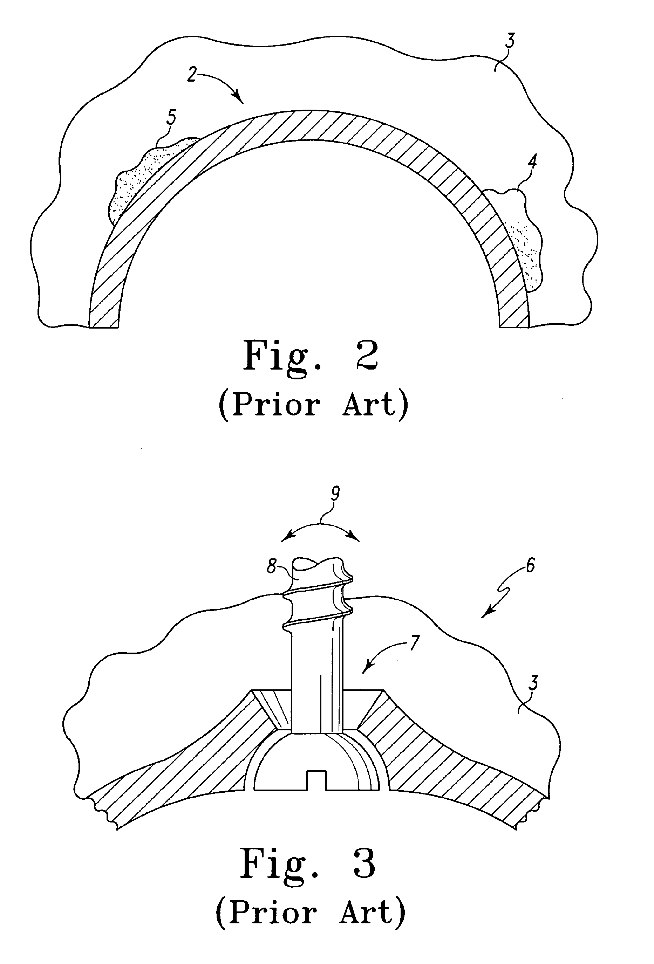

Referring now to FIG. 2, a prior art hip cup 2 is shown. The hip cup 2 is secured to acetabulum 3 of the patient. The hip cup 2 may become loose from the acetabulum particularly if the acetabulum 3 includes portions 4, which have weak bone, or portions 5, which represent voids or missing bone to support the hip cup 2.

The hip cup 6 of FIG. 3 includes an opening 7 for receiving a screw 8. The screw 8 is free to move in the direction of arrows 9 and thus does not rigidly secure the hip cup 6 to acetabulum 3.

The present invention provides for an orthopaedic implant reconstruction device, which includes fasteners that mechanically lock with the acetabular device to establish a fixed angle between the orthopaedic implant and the fastener.

The orthopaedic reconstruction device of the present invention includes multiple aspects such as a fastener in the form of a screw that locks into the orthopaedic implant. The fastener may be in the form of a screw, for example, a cancellous screw that is secured to cancellous bone. Alternatively, the screw may be in the form of a cortical screw that is secured to cortical bone. The screw may lock into a central portion of the implant or to an outer portion, for example, a rim of the orthopaedic implant. For example, the screw may be placed in the rim of a flanged orthopaedic hip cup.

The locking orthopaedic implant of the assembly may include a screw that locks to the acetabular reconstruction device in a single orientation relative to the device. Alternatively, the fastener may be in the form of a screw or a pin that may lock to the reconstruction device in any one of infinite orientations with respect to the device. Such variable orientations of a locking fastener may be accomplished by one of many devices such as by using polyaxial bushing technology. The polyaxial bushing has a generally spherical outer diameter "OD" and a tapered cylindrical inner diameter "ID." The bushing is split readily so that it may compress upon the insertion of a fastener through the tapered bore. The fastener expands the tapered bore causing the spherical periphery of the bushing to lockably engage with the spherical bore on the orthopaedic implant.

The orthopaedic implant locking fastener assembly may contain a locking fastener that contains no threads. The non-threaded portion may be in the form of a cone, a cylinder or similar feature that may include bone ingrowth surface treatments or ingrowth surface coatings that may improve the adherence of bone to the fastener. It should be appreciated that the locking screw orthopaedic implant assembly of the present invention may include a combination of any of the aforementioned components.

The present invention includes an orthopaedic joint reconstruction component that utilizes a fastener that is mechanically locked to the component. The fastener mechanically locks to the device by, for example, the use of threads or by a polyaxial bushing positioned between the component and the fastener. The fastener may attach to the orthopaedic implant in the central portion of the implant or along the rim of the implant or flange of the implant. The fastener may be secured to another device that is connected to the orthopaedic implant component. The fastener attaches the orthopaedic implant to bone. The fastener remains mechanically locked to the orthopaedic implant such that it becomes a mechanical extension of the orthopaedic implant allowing it to support the orthopaedic implant if bone behind the orthopaedic implant begins to resorb.

While this device is well suited for any joint component of an orthopaedic reconstruction implant, it should be appreciated that cups or shells of a hip reconstruction joint are particularly well suited for the use of locking fasteners. It should also be appreciated that locking fasteners in the form of screws, either cancellous screws to connect with cancellous bone or cortical screws to connect with cortical bone are particularly well suited for this invention. It should also be appreciated that the locking orthopaedic implant joint component fastener assembly of the present invention may be well suited for other orthopaedic joint components besides an orthopaedic cup or shell, for example, a tibia tray, or a shoulder glenoid component. It should also be appreciated that any orthopaedic joint component, which may have a need for additional securing may utilize the locking fastener of the present invention.

In one aspect, the invention provides an acetabular cup assembly for attachment to an acetabulum. The cup includes a body having an interior wall and defining an opening in the body. The cup also includes a fastener having a portion thereof fitted through the opening of the body. The fastener can be rigidly connected to the body. The fastener can be connected to the body in a selected one of a plurality of angular positions relative to the body.

In another aspect, the invention provides a kit for performing joint arthroplasty. The kit includes a joint component having an interior wall defining an opening in the body and a fastener having a portion thereof fitted through the opening of the body. The fastener can be rigidly connected to the body. The fastener can be connected to the body in a selected one of a plurality of angular positions relative to the body.

The devices which are provided by the invention can be used in a method of performing joint arthroplasty on a bone of a patient. The method includes the steps of providing a first joint component. The first joint component has at least one plate with holes through the joint component and a bushing movably coupled in the plate hole. The first joint component also has a radially exterior surface, an opposite interior surface, and first and second ends defining a passageway therebetween. The first joint component also has an attachment component for cooperation with the bushing sized for extension into the passageway. Each attachment component includes opposed leading and trailing portions. The method also includes the step of positioning the first joint component upon the bone portions so that the plate hole in the first joint component is situated over bone. The method also includes the steps of inserting the attachment component into the passageway and aligning the attachment component so that it is aligned toward bone suitable for attachment. The method also includes the step of seating the attachment component to the first joint component.

In a further aspect, the invention provides an orthopaedic joint assembly for attachment to a bone. The joint assembly includes a body having an interior wall defining an opening in the body and a fastener. The fastener has a portion of the fastener fitted through the opening of the body. The fastener is rigidly connected to the body.

In yet another aspect, the invention provides a kit for performing joint arthroplasty. The kit includes a joint component having an interior wall defining an opening in the body and a fastener having a portion of the fastener fitted through the opening of the body. The fastener is rigidly connected to the body.

In a yet further aspect, the invention provides a method for performing joint arthroplasty on a bone of a patient. The method includes the step of providing a first joint component. The first joint component has at least one hole through the joint component, a bushing movably coupled in the hole and having a radially exterior surface, an opposite interior surface, and first and second ends defining a passageway between the first and second ends. The method also includes the step of providing an attachment component for cooperation with the bushing of the first joint component and sized for extension into the passageway. The attachment component includes opposed leading and trailing portions. The method also includes the steps of positioning the first joint component upon the bone portions so that the hole in the first joint component is situated over bone and inserting the attachment component into the passageway. The method further includes the step of aligning the attachment component so that it is aligned toward bone suitable for attachment and seating the attachment component to the first joint component.

In another aspect, the invention provides an acetabular cup assembly for attachment to an acetabulum. The cup assembly includes an acetabular cup including a first connector and a fastener. The fastener cooperates with the cup. The fastener includes a second connector. The first and second connectors cooperate to rigidly connect the fastener to the cup.

The invention also provides a fastener assembly for use to secure an acetabular cup to an acetabulum. The fastener assembly includes a fastener. The fastener includes a fastening portion for cooperation with the acetabulum and a connecting portion. The fastener assembly also includes a bushing including a generally spherical outer periphery and an interior wall defining a central opening through the bushing. The interior wall cooperates with the connecting portion of the fastener.

The invention further provides a fastener for use to secure an acetabular cup to an acetabulum. The fastener includes a fastening portion for cooperation with the acetabulum and a connecting portion for connection with the cup.

Technical advantages of the present invention include the ability to mechanically lock the screw to the orthopaedic implant. For example, according to an aspect of the present invention, an orthopaedic joint assembly for attachment to a bone is provided. The orthopaedic joint assembly includes a body having an interior wall defining an opening in the body. The orthopaedic joint assembly further includes a fastener having a portion of the fastener fitted through the opening of the body. The fastener is rigidly connected to the body. Thus, the present invention provides the ability to mechanically lock the screw to the orthopaedic implant component.

The technical advantages of the invention also include the ability to provide better fixation if significant defects are found in the bone adjoining the orthopaedic joint implant. For example, according to another aspect of the present invention, an orthopaedic joint assembly for attachment to a bone is provided. The joint assembly includes a body having an anterior wall defining an opening in the body. The joint assembly also includes a fastener having a portion of the fastener fitted through the opening of the body. The fastener is rigidly connected to the body. Thus the present invention provides for better fixation if significant defects occur by permitting the fastener to better support the body by being rigidly attached to the body.

The technical advantages of the invention also include an ability to provide better fixation to poor quality bone. For example, according to another aspect of the present invention, an orthopaedic joint assembly for attachment to bone is provided. The joint assembly includes an orthopaedic joint component having an interior wall defining an opening in the body. The joint assembly also includes a fastener having a portion fitted through the opening of the orthopaedic joint component. The fastener is rigidly connected to the orthopaedic joint component. Thus the present invention provides for better fixation of the orthopaedic implant component to bone when poor quality bone is positioned near the joint.

The technical advantages of the present invention also include the ability to provide more options to achieve bone fixation in sub-optimum bone conditions, where the patient may have good bone quality in certain locations and poor bone quality in other locations. For example, according to another aspect of the present invention, an orthopaedic joint component is provided for attachment to a bone. The orthopaedic joint component has an interior wall defining an opening in the component. The joint assembly also includes a fastener having a portion rigidly connected to the body in a selected one of a plurality of angular positions relative to the orthopaedic component. Thus the present invention provides for more options to achieve bone fixation by permitting the fastener to be rigidly positioned in one of several positions in order that the fastener is aligned with better quality bone rather than with poor quality bone.

The technical advantages of the present invention also include the ability to keep the orthopaedic joint component stable. For example, according to another aspect of the present invention, an orthopaedic joint assembly for attachment to bone is provided. The joint assembly includes an orthopaedic joint component having an interior wall defining a component opening in the component. The joint assembly also includes a fastener having a portion of the fastener fitted through the opening of the component. The fastener is rigidly connected to the component. Thus the present invention provides for a rigid fastener to keep the acetabular device stable in the bone.

The technical advantages of the present invention further include the ability to compensate for the resorption of bone over time behind the orthopaedic joint component. For example, according to another aspect of the present invention, an orthopaedic joint assembly is provided for attachment to a bone. The joint assembly includes a joint component having an interior wall defining an opening in the joint component. The joint assembly also includes a fastener having a portion of the fastener fitted through the opening of the component. The fastener is rigidly connected to the component providing a rigid construct that provides support for the orthopaedic joint component when resorption of bone around the component occurs.

Embodiments of the invention will now be described by way of example with reference to the accompanying drawings, in which:

FIG. 1 is a plan view partially in cross-section of a hip cup and screw assembly in accordance with another embodiment of the present invention;
FIG. 1A is a plan view partially in cross-section of a modular hip cup with three spikes in accordance with an embodiment of the present invention;
FIG. 1B is a partial plan view, partially in cross section of a modular hip cup with pegs with through openings for the pegs according to another embodiment of the present invention;
FIG. 1C is a partial plan view, partially in cross section of a modular hip cup with pegs with tapered openings for the pegs according to another embodiment of the present invention;
FIG. 1D is a top view of a modular hip cup with equally spaced-apart pegs according to another embodiment of the present invention;
FIG. 2 is a cross-sectional view of a prior art hip cup;
FIG. 3 is a cross-sectional view of a prior art hip cup assembly including a screw;
FIG. 4 is a cross-sectional view of a hip cup assembly including a locking screw according to another embodiment of the present invention;
FIG. 4A is a partial plan view, partially in cross-section of FIG. 4 showing the tapered threaded connection in greater detail;
FIG. 4B is a partial plan view, partially in cross-section of FIG. 4 showing the threaded tapered opening in greater detail;
FIG. 5 is a plan view, partially in cross-section of a hip prosthesis assembly including the hip cup assembly of FIG. 4;
FIG. 5A is a partial plan view, partially in cross-section of FIG. 5 showing the flange connection in greater detail;
FIG. 6 is a partial plan view partially in cross-section of the hip cup assembly of FIG. 4;
FIG. 7 is a plan view of the locking screw of the hip cup assembly of FIG. 4;
FIG. 7A is a partial plan view, partially in cross-section of a hip cup assembly showing a coated peg according to another embodiment of the present invention;
FIG. 8 is a perspective view partially in cross-section of a hip cup assembly including a pivotable locking screw according to yet another embodiment of the present invention;
FIG. 9 is a partial plan view partially in cross-section of a hip prosthesis assembly including the hip cup assembly of FIG. 8;
FIG. 10 is a plan view partially in cross-section of the hip prosthesis assembly of FIG. 9 in position in a femur;
FIG. 11 is a partial plan view, partially in cross-section, of the hip cup assembly of FIG. 8;
FIG. 12 is a plan view of the locking screw of the hip cup assembly of FIG. 8;
FIG. 13 is a top view of the bushing of the hip cup assembly of FIG. 8;
FIG. 14 is a plan view of the bushing of FIG. 13;
FIG. 15 is a plan view partially in cross-section of a hip cup assembly including a plurality of pivotable locking screws according to yet another embodiment of the present invention;
FIG. 15A is a top view of a modular hip cup assembly with three equally spaced-apart screws according to another embodiment of the present invention;
FIG. 16 is a plan view, partially in cross-section, of a hip cup assembly including a plurality of rigid locking screws according to yet another embodiment of the present invention;
FIG. 16A is a top view of a hip cup assembly with four equally spaced-apart screws according to another embodiment of the present invention;
FIG. 17 is a partial plan view, partially in cross-section, of the hip cup assembly of FIG. 16;
FIG. 17A is a partial plan view, partially in cross-section, of a hip cup assembly including the cup of the hip cup assembly of FIG. 16 and a plurality of non-rigid screws according to yet another embodiment of the present invention;
FIG. 18 is a partial top view, partially in cross-section, of the hip cup assembly of FIG. 16;
FIG. 18A is a partial top view, partially in cross-section, of the hip cup assembly of FIG. 17A;
FIG. 19 is a plan view, partially in cross-section, of a hip cup assembly including a plurality of rigid locking screws, according to yet another embodiment of the present invention;
FIG. 19A is a top view of a hip cup assembly with four equally spaced-apart screws, according to another embodiment of the present invention;
FIG. 20 is a plan view, partially in cross-section, of a hip screw assembly with a tapered locking screw, according to another embodiment of the present invention;
FIG. 21 is a plan view of a hip cup assembly including a hip cup having a plurality of flanges with holes for receiving rigid locking screws, according to yet another embodiment of the present invention;
FIG. 21A is a cross-sectional view of FIG. 21 along the lines 21A-21A in the direction of the arrows;
FIG. 22 is a cross-sectional view of a hip cup assembly similar to FIG. 21 having pivotal locking screws;
FIG. 23 is a plan view, partially in cross-section, of a hip cup assembly including a rigid locking screw having a tapered connection portion and an adjacent securing screw, according to yet another embodiment of the present invention;
FIG. 23A is a partial top view of the hip cup assembly of FIG. 23 showing the screws in greater detail;
FIG. 23B is a partial plan view, partially in cross-section, of the hip cup assembly of FIG. 23 showing the screws in greater detail;
FIG. 23C is a partial top view of a hip cup assembly including a rigid locking screw having a tapered connection portion and an adjacent securing screw with the tapered screw having a cut out portion for receiving the securing screw, according to yet another embodiment of the present invention;
FIG. 23D is a partial plan view of a hip cup assembly including a rigid locking screw having a tapered connection portion and an adjacent securing screw with the securing screw overlying the tapered screw, according to yet another embodiment of the present invention;
FIG. 24 is a plan view, partially in cross-section, of a hip cup assembly including a cup and a plurality of screws including rigid screws and non-rigid screws, according to yet another embodiment of the present invention;
FIG. 25 is a partial plan view, partially in cross-section, of a knee prostheses including a knee tibial implant for use in performing knee orthopaedic surgery including rigid screws, in accordance with yet another embodiment of the present invention;
FIG. 26 is a plan view, partially in cross-section, of a shoulder prostheses including a glenoid implant for use in performing shoulder orthopaedic surgery including rigid screws, in accordance with yet another embodiment of the present invention;
FIG. 27 is a plan view of a kit for use in performing hip surgery, in accordance with yet another embodiment of the present invention;
FIG. 28 is a plan view of a kit for use in performing hip surgery, in accordance with yet another embodiment of the present invention;
FIG. 29 is a flow diagram of a method of performing orthopaedic surgery, in accordance with yet another embodiment of the present invention; and
FIG. 30 is a flow diagram of another method of performing orthopaedic surgery, in

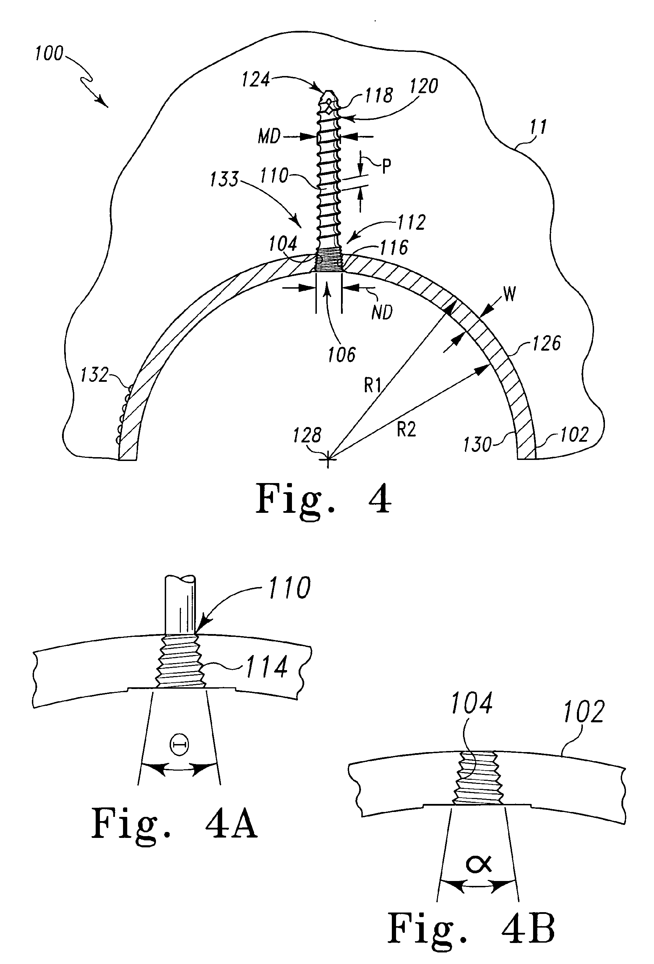

Referring to the drawings, FIG. 4 shows an orthopaedic implant joint assembly 100 such as, for example, a hip stem, a femoral knee component, a tibial knee, a humeral shoulder, a glenoid shoulder, a wrist component, elbow component, or an ankle component. It should be appreciated that the orthopaedic joint assembly may be an assembly of any orthopaedic implant joint component and a fastener.

The orthopaedic joint assembly 100 includes a body 102. The body 102 may be in the form of any orthopaedic joint component. For example, the body 102 may be a hip stem, a hip cup, a knee femoral component, a knee tibial component, a shoulder glenoid component, a shoulder humeral component or any component that represents a part of an orthopaedic joint assembly.

The body 102 as shown in Fig. 4, is in the form of a hip cup or shell. The body 102 has an interior wall 104 formed in the body 102. The interior wall 104 defines an opening 106 in the body 102.

The orthopaedic joint assembly 100 further includes a fastener 110. The fastener 110 includes a connecting portion 112 of the fastener 110. The connecting portion 112 is fitted through the opening 106 of the body 102. The fastener 110 is rigidly connected to the body 102. The fastener 110 may be any fastener capable of rigid connection with the body 102.

For example and as shown in FIG. 4, the fastener 110 may be in the form of a screw. For example, the fastener 110 may include external threads 114. The external threads 114 may be positioned on the connecting portion 112 of the fastener 110 that is fitted through the opening 106 of the body 102. The external threads 114 of the fastener 110 may, as shown in FIG. 4, cooperate with internal threads 116 formed on the interior wall 104 of the body 102.

The external threads 114 of the fastener 110 and the internal threads 116 of the body 106 may, as shown in FIG. 4, be matingly fitted to provide for a rigid construction of the fastener 110 to the body 102. While the external threads 114 on the fastener 110 and the internal threads 116 of the body 102 may be straight or cylindrical, to assure the rigidity of the fastener 106 to the body 102, the threads may be tapered.

For example, as shown in FIG. 4A, the external threads 114 of the fastener 110 may form an angle $\theta$. The external threads 14 may form an angle $\theta$ of, for example, 2 to 14°. For example, the angle $\theta$ may be about 4 to 6°.

Referring now to FIG. 4B, the interior wall 104 of the body 102 may form an included angle $\alpha$ of, for example, an angle equal to the angle $\theta$ of the external threads 114 of the fastener 110 (see FIG. 4A).

Referring again to FIG. 4, to provide for rigid construction between the fastener 110 and the body 102, the external threads 114 of the fastener 110 and the internal threads 116 of the body 102 are designed for optimum strength. The external threads 114 and the internal threads 116 may, to optimize the strength, be made of a fine pitch. Further, the external threads 114 and the internal threads 116 preferably mate with each other and may, as shown in FIG. 4, comprise multiple thread leads. For example, the external threads 114 may be double or triple leads. It should be appreciated the external threads 114 may be quadrupled or greater multiple threads.

The fastener 110 may have a second thread or bone thread 118 on second portion 120 of the fastener 110 that may be threadably installed through the opening 106 in the body 102. As shown in FIG. 4, the pitch of the threads 118 on the second portion 120 may be the same as the pitch of the internal threads 116 of the body 102. If the major diameter MD of the threads 118 is less than minor diameter ND of the internal threads 116, the second portion 120 of the fastener 110 may freely pass through opening 106 of body 102. By providing such multiple threads on the external threads 114 and on the internal threads 116, and by having the same pitch in the bone threads 118, a strong screw thread configuration may be provided for the fastener 110.

As shown in FIG. 4, the bone threads 118 may be straight or not tapered. The bone threads 118 may be adapted for use with cancellous bone or cortical bone. As shown in FIG. 4, the bone threads 118 are adapted for use with soft or cancellous bone. The fastener 110 may, as shown in FIG. 4, include an end or tip 124 that provides for self-drilling and/or self tapping, such that a drill and/or tap are not required to prepare the passageway for the fastener 110.

The body or prosthetic component 102 may, as shown in FIG. 4, be in the form of a hip cup. The hip cup 102 may be defined as a sector of a sphere defining a convex external periphery 126 defined by radius R1 extending from origin 128. The cup 102 is further defined by a concave inner-periphery 130 defined by radius R2 extending from origin 128. The hip cup 2 thus has a wall thickness W, which as shown in FIG. 4, is generally uniform.

The hip cup 102 may include a porous coating 132 applied to the convex external periphery 126.

The fastener 110 may, as shown in FIG. 4, be positioned in apex or zenith 133 of the hip cup 102 as shown in FIG. 4. The fastener 110 may extend perpendicularly from the convex external periphery 126 as shown in FIG. 4. It should be appreciated alternatively that the screw fastener 110 may be positioned obliquely or at a acute angle with respect to the periphery 126.

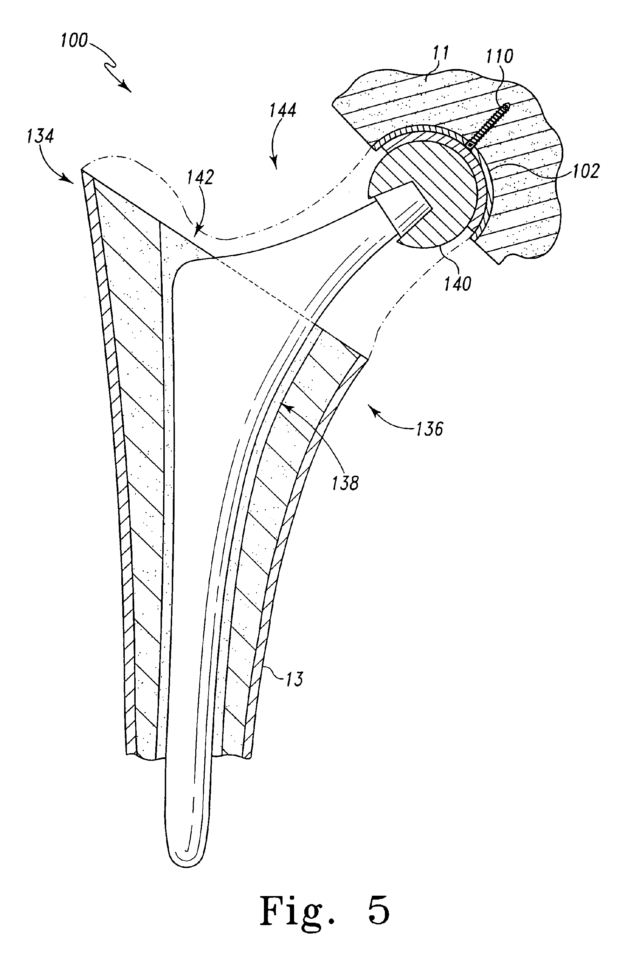

Referring now to FIG. 5, the hip cup assembly 100 of FIG. 4 is shown implanted in hip prosthesis 134. The hip prosthesis 134 includes the hip cup or shell 102, which is secured to acetabulum 11 by fastener or screw 110. The hip prosthesis 134 further includes a hip stem assembly 136 including a stem 138 to which a ball or head 140 is removably secured. The stem 138 is fitted into cavity 142 formed in the medullary canal 144 of femur 13.

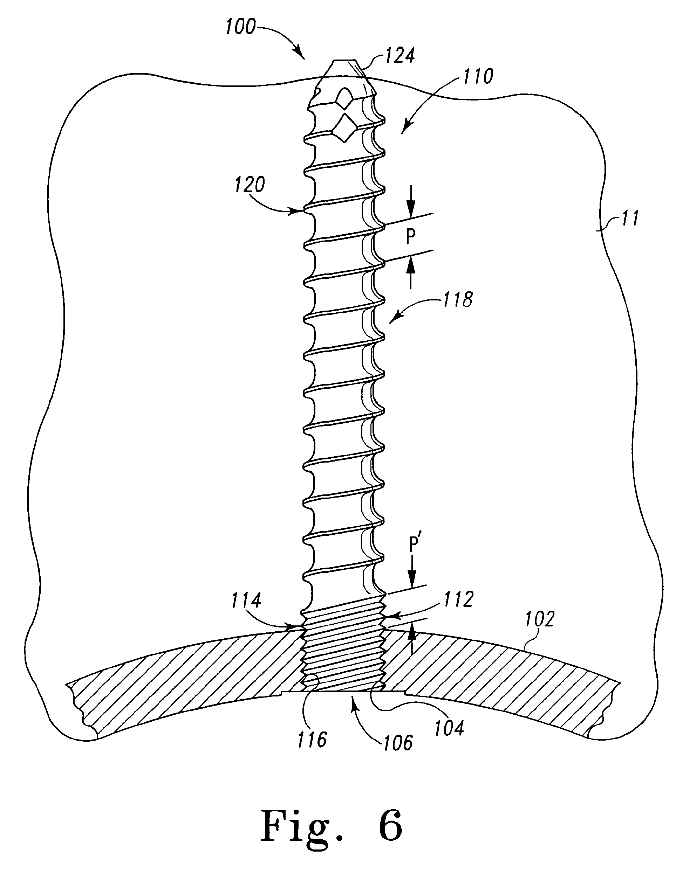

Referring now to FIG. 6, the fastener 110 is shown in greater detail assembled to the cup 102 to form the hip cup assembly 100. As shown in FIG. 6, the external threads 114 on the first portion or connecting portion 112, has a triple lead where three adjacent threads form a pitch P' representing a pitch of the triple lead thread 114. The second portion or fastening portion 120 of the screw 110 includes bone threads 118 defined by pitch P representing the distance between adjacent threads 118.

It should be appreciated that the pitch P and the pitch P' of the external threads 114 may be the same. By providing the pitch P and the pitch P' with the same pitch, the bone threads 118 may be threaded through the internal threads 116 of the cup 102 until the screw 110 is in its engaged, rigid position, such that connecting portion 112 of the screw 110 engages with the cup 102 and the external threads 114 of screw 110 engage with the internal threads 116 of cup 102.

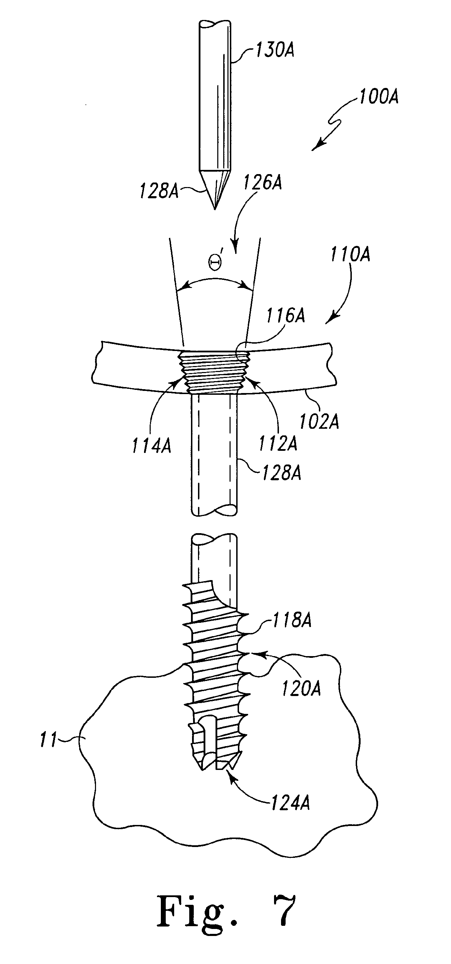

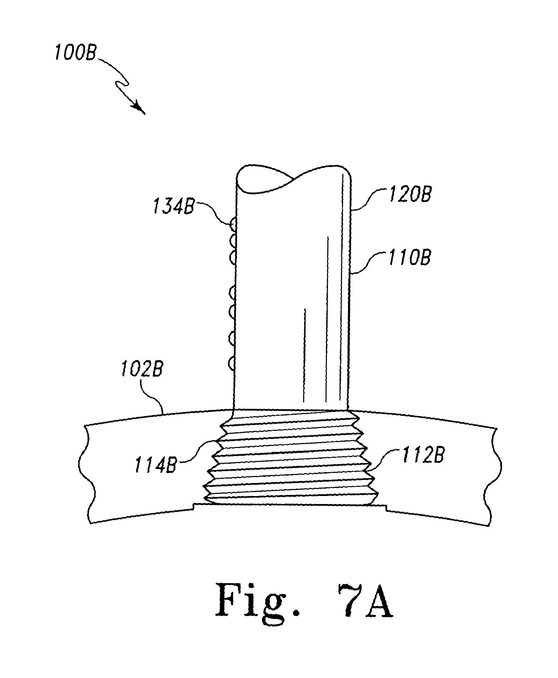

Referring now to FIG. 7, an alternate embodiment of the present invention is shown as hip cup assembly 100A. Hip cup assembly 100A includes hip cup 102A, which is similar to the hip cup 102 of FIGS. 4, 5 and 6. The hip cup assembly 102A further includes a cannulated screw 110A. The cannulated screw 110A includes a central axial opening or cannula 126A. The cannulated screw 110A includes a first connecting portion 112A, which includes tapered threads 114A, which mate with internal tapered threads 116A formed in the cup 102A.

The external threads 114A as shown in FIG. 7, are tapered and defined by an included angle θ'. The tapered threads 114A may be similar to the threads 114 of FIG. 6. The screw 110A may further include a fastening portion 120A, including cancellous bone threads 118A. The screw 110A may further include a smooth shanked portion 128A, positioned between the connecting portion 112A and the fastening portion 120A. The cannulated screw 110A may further include a self-drilling and self-tapping tip 124A extending from the fastening portion 120A.

The cannula or opening 126A of the cannulated screw 110A is adapted to receive pin 130A, which slidably fits within the canula 126A of the cannulated screw 110A. The pin 130A may include a tip 128A for engagement with acetabulum 11. The pin 130A may be inserted before or after the cup 102A is installed on the acetabulum 11. If the pin 130A is installed first, the pin 130A may serve to guide the screw 110A into position. If the pin 130A is installed later, the pin 130A may extend beyond the connecting portion 112A of the cannulated screw 110A, and may serve for positioning a stem (see FIG. 5).

Referring now to FIG. 7A, yet another embodiment of the present invention is shown as hip cup assembly 100B, which is similar to the hip cup assembly 100 of FIGS. 4, 5 and 6, except that the connector 110 of FIGS. 4, 5 and 6 is replaced with peg 110B. The peg 110B is lockably engaged with cup 102B. The peg 110B includes a fastening portion 120B, which is generally cylindrical and smooth, and a connecting portion 112B, which includes external tapered threads 114B, similar to the threads 114 of FIGS. 4, 5 and 6. The peg 110B may include a bone growth-enhancing feature, for example, a porous coated surface treatment 134B.

While it should be appreciated that the fastener of the present invention, for example, fastener 110 of the hip cup assembly 100, may include threads such as bone threads 118, it should be appreciated that the fastener may not include threads or may be smooth or, for example, may be generally cylindrical. For example, the fastener may be in the form of peg 110B of FIG. 7A. It should be appreciated that the pegs, which serve as fasteners for the present invention, may be installed into cancellous bone or may be fitted into pre-cut or pre-drilled and/or pre-reamed openings. It should further be appreciated that the pegs may be put in position in the acetabulum after the hip cup or other implant is implanted or pre-assembled to the hip cup or implant and then installed with the orthopaedic implant.

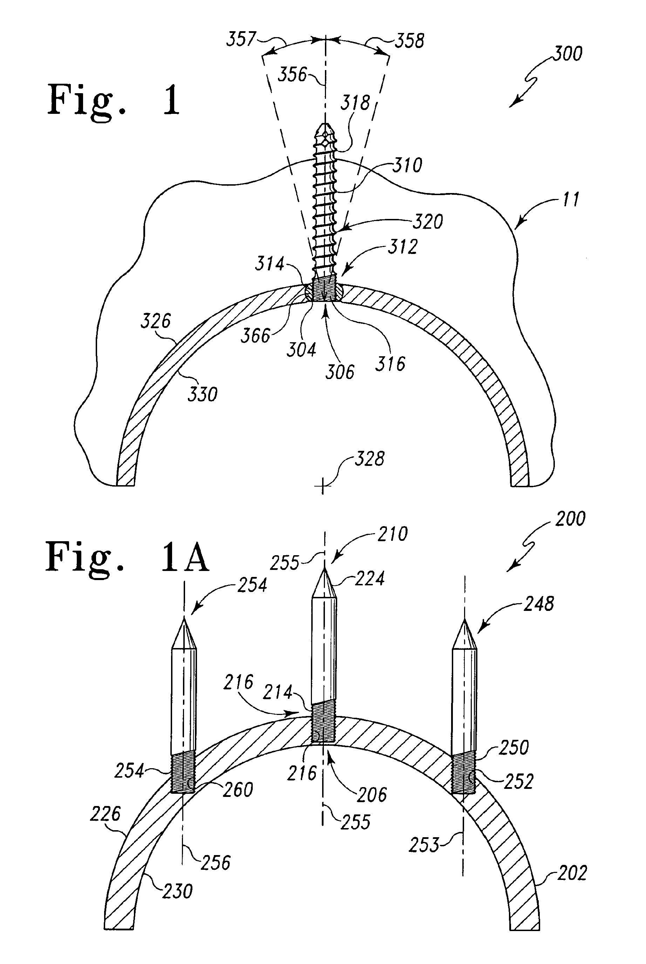

According to the present invention, and referring now to FIG. 1A, an embodiment of the present invention is shown as hip cup assembly 200, in which pegs are included with the hip cup to form hip cup assembly 200. The assembly 200 is installed as a unit into the acetabulum 11.

The hip cup assembly 200 includes a hip cup 202, which is similar to hip cup 102 of FIG. 4. Hip cup 202 includes a convex external periphery 226 and a concave internal periphery 230. Hip cup assembly 200 further includes a first fastener 210 in the form of a generally cylindrical pin. The pin 210 includes a tip 224 that may, as shown in FIG. 1A, have a generally conical shape. The pin 210 is connected to the cup 202 by, for example, a connecting portion 212 that fits within opening 206 formed in the hip cup 202.

As shown in FIG. 1A, the pin 210 may be secured to the hip cup 202 by, for example external threads 214, which mate with internal threads 216 formed in opening 206 of the hip cup 202. As shown in FIG. 1A, the external threads 214 and the internal threads 216 may be cylindrical. It should be appreciated that the external threads 214 and the internal threads 216 may be tapered.

While the hip cup assembly 200 may have a solitary pin 210, it should be appreciated that additional pins may be utilized. For example, as shown in FIG. 1A, the hip cup assembly 200 includes a second fastener 248 is similar to the first fastener 210. The second fastener 248 is secured by external threads 250 to internal threads 252 formed in the hip cup 202.

To permit the hip cup assembly 200 to be installed into an acetabulum in the assembled state, the second fastener 248 includes a longitudinal centerline 253, which is parallel to longitudinal centerline 255 of the first fastener 210. In other words, the second fastener 248 and the first fastener 210 are parallel and spaced from each other.

The hip cup assembly 200 may further include a third fastener 254, which is similar in shape and size to the first and second fasteners 210 and 248. The third fastener 254 defines a third fastener centerline 256, which is parallel to the first fastener centerline 255. The third fastener 254 defines external threads 258, which match with internal threads 260 formed in the hip cup 202. As shown in FIG. 1A, the opening, for example, the first opening 206, may not extend completely through the hip cup 202. When the opening 206 does not extend through the hip cup 202, the fasteners, for example, first fastener 210 is inserted from the external convex periphery 226 of the hip cup 202.

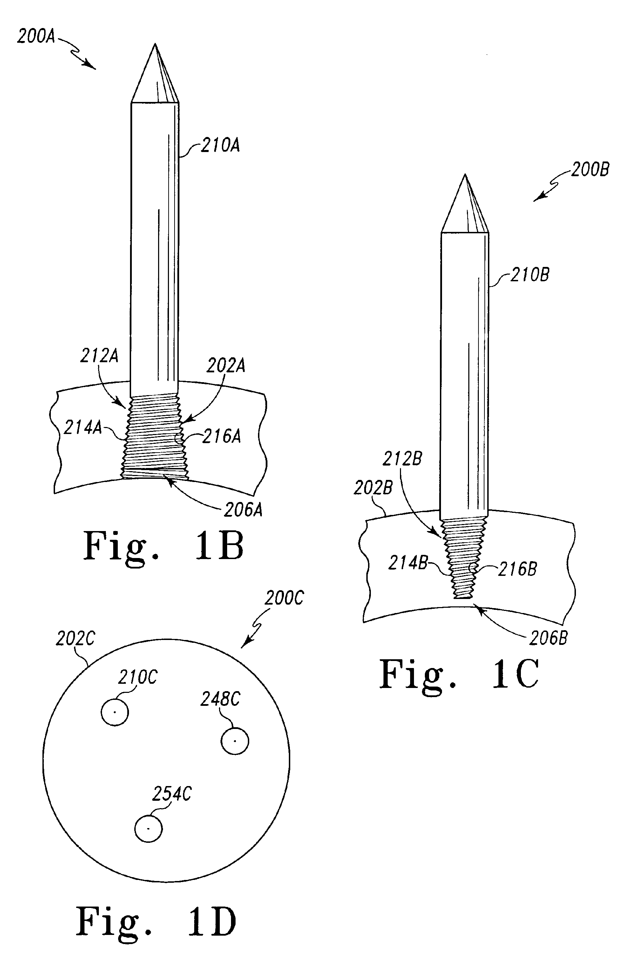

The pins for the hip cup assembly of the present invention may include pins with alternative connecting constructions. For example, and as shown in FIG. 1B, a hip cup assembly 200A according to the present invention is shown. The hip cup 200A includes a cup 202A similar to the cup 202 of FIG. 1A, as well as a pin 210A. The pin 210A is somewhat similar to the pin 210A of FIG. 1A, except that the pin 210A includes a connecting portion 212A, which is tapered. The tapered connecting portion 212A includes external threads 214A, which mate with internal threads 216A formed on the hip cup 202A. It should be appreciated that the hip cup assembly 200A includes an opening 206A, which extends through the cross section of the cup 202A.

As shown in FIG. 1C, the hip cup assembly of the present invention may be in the form of hip cup assembly 200B. The hip cup assembly 200B is similar to the hip cup assembly 200 of FIG. 1A, except the hip cup assembly 200B includes a pin 210B, which is somewhat different than the pin 210A of the hip cup assembly 200 of FIG. 1A. The pin 210B includes a connecting portion 212B, which is tapered in the opposing direction to the connecting portion 212A of the pin 210A of FIG. 1B. The connecting portion 212B includes external threads 214B, which mate with internal threads 216B formed on hip cup 202B. It should be appreciated that the hip cup 202B includes an opening 206B, which may, but does not need to be, a through hole.

While the fasteners of the hip cup may be as shown in FIG. 1A, in a single plane, it should be appreciated that the fasteners may be equally spaced around the hip cup. For example, and as shown in FIG. 1D, the orthopaedic joint assembly of the present invention may be in the form of hip cup assembly 200C, which includes a hip cup 202C and three equally spaced fasteners, which may be in the form of pegs. The fasteners or pegs may include the first fastener 210C, second fastener 248C and the third fastener 254C.

It should be appreciated that an orthopaedic joint assembly according to the present invention can be provided with locking fasteners that lock in a particular direction. It should be appreciated that such a locked configuration may not be particularly well suited for bones with osteoporadic conditions or for patients with bone voids. The fixed location of a fixed locked fastener may be in alignment with the void or the soft bone and may not correspond to where the bone of that particular patient is best. Therefore, the locked prosthetic joint assembly of the present invention may include a bone fastener that may be positioned in a multitude of angular positions within the bone.

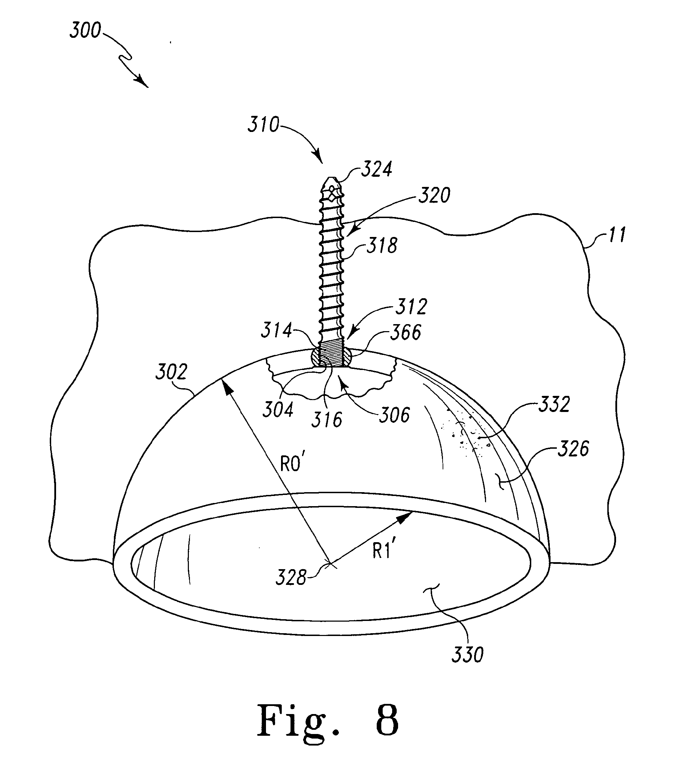

According to the present invention, and referring now to FIG. 8, a prosthetic joint assembly, including a positionable fastener, is shown as prosthetic joint assembly or hip cup assembly 300. The hip cup assembly 300 includes a hip cup 302 having a generally hemispherical shape and defined by a convex outer periphery 326 defined by radius R0' extending from origin 328. The hip cup 302 is further defined by a concave inner periphery 330 extending a radius R1' from origin 328. The hip cup 302 includes an internal wall 304, which defines opening 306 through the cup 302.

The orthopaedic joint assembly 300 further includes a fastener 310 in the form of, for example, a screw. The fastener 310 includes a connecting portion 312 fitted through the opening 306 of the cup 302. The fastener 310 is rigidly connected to the cup 302. The fastener 310 as shown in FIG. 8, may be rigidly connected to the cup 302 in one of a plurality of angular positions relative to the cup 302.

The ability to angularly adjust the position of the fastener 310 in the cup 302 may be accomplished in many different ways. For example and as shown in FIG. 8, an additional component may be positioned between the cup 302 and the fastener 310 to provide for the angular positioning capability of the prosthetic joint assembly 300.

For example and as shown in FIG. 8, a bushing 366 may be positioned between the fastener 310 and the cup 302. As shown in FIG. 8, the fastener 310 may include the connecting portion 312 for connecting with the bushing 366. The connecting portion 312 may include external threads 314 for cooperating with internal threads 316 formed in the bushing 366. The fastener 310 may further include a fastening portion 320, which may include bone threads 318 for engagement with the acetabulum 11. The fastener 310 may include a tip 324. The tip 324 may include self-drilling and/or self-tapping features.

The cup 302 may include a porous coating 332 for assisting in bone ingrowth between the cup 302 and the acetabulum 11 to enhance fixation of the cup 302 to the acetabulum 11.

Referring now to FIG. 1, the hip cup assembly 300 is shown with the fastener 310 in a first position with the fastener 310 aligned with first centerline 356 of the fastener 310. It should be appreciated that the fastener 310 may be able to move about to form a conical path of permitted positions extending from the first central centerline 356 to a second end centerline 357 and to an opposed third end centerline 358. By permitting the fastener 310 to be locked in any position from longitudinal centerline first central position 356 to the longitudinal centerline end positions 357 and 358, a wide variety of fastener positions may be engaged by the fastener 310 to match with preferred bone locations of the patient.

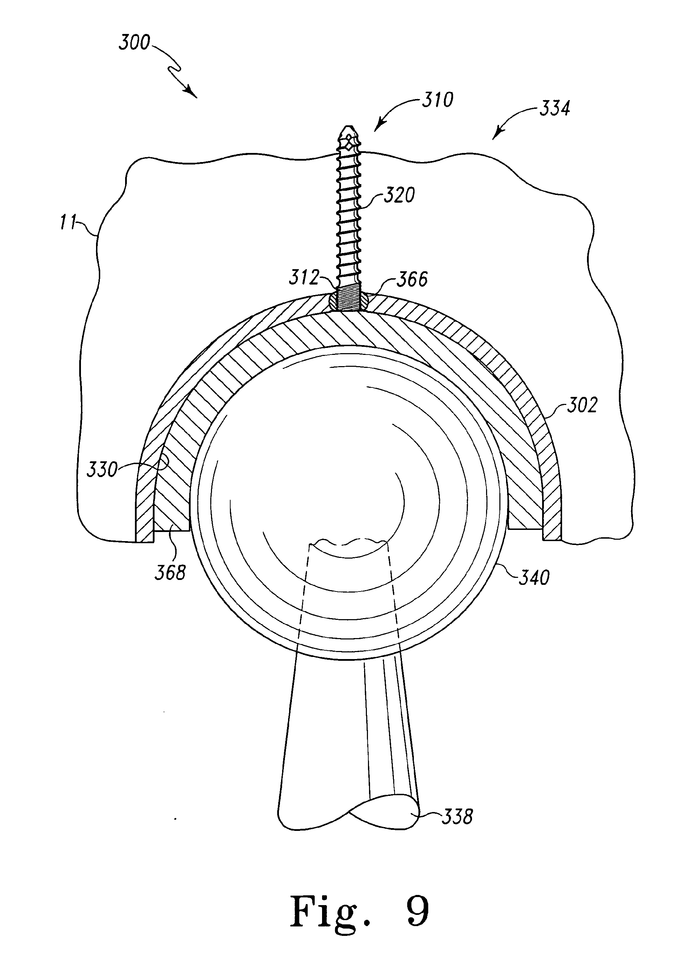

Referring now to FIG. 9, hip cup assembly 300 is shown as part of hip prosthesis 334. The hip cup assembly 300 includes hip cup 302 to which bushing 366 is secured. Fastener 310 is lockably secured by the bushing 366 to hip cup 302. The hip prosthesis 334 further includes a hip stem 338 to which head 340 is, as shown as FIG. 9, removably secured. The head 340 may directly articulate with inner periphery 330 of the hip cup 302 or may, as shown in FIG. 9, include a bearing or liner 368, which is positioned between the hip cup 302 and the head 340. The bearing 368 may be made of any suitable material, for example, a ceramic, a metal or a plastic. The bearing 368 may be made of polyethylene, for example, ultra-high molecular weight polyethylene.

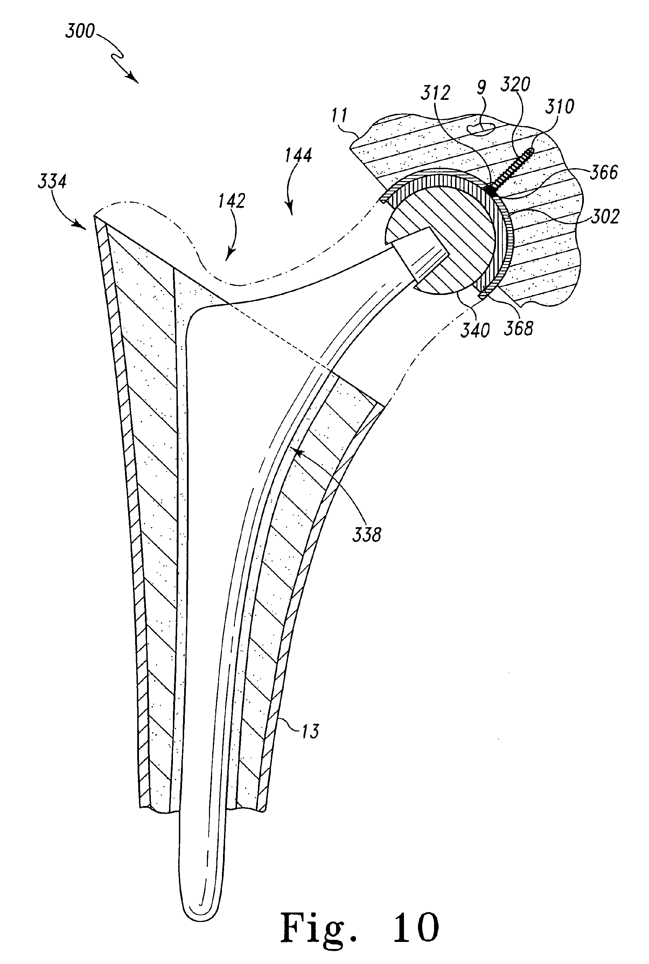

Referring now to FIG. 10, the hip prosthesis 334 is shown in a patient. The hip prosthesis 334 includes the hip stem 338 fitted into cavity 142 formed intramedullary canal 144 of femur 13. Head 340 is fixably removably secured to hip stem 338. A bearing 368 is pivotally positioned over head 340 as well as positioned within cup 302. The cup 302 contains bushing 366, which secures fastener 310 fixably to the cup 302. The fastener is secured to acetabulum 11. It should be appreciated that the fastener 310 in the position as shown in FIG. 10, is capable of reaching good healthy bone in the acetabulum and avoid positions of the acetabulum that may include voids or weak cancellous osteoporatic bone 9 which may not provide a good location for the fastener 310.

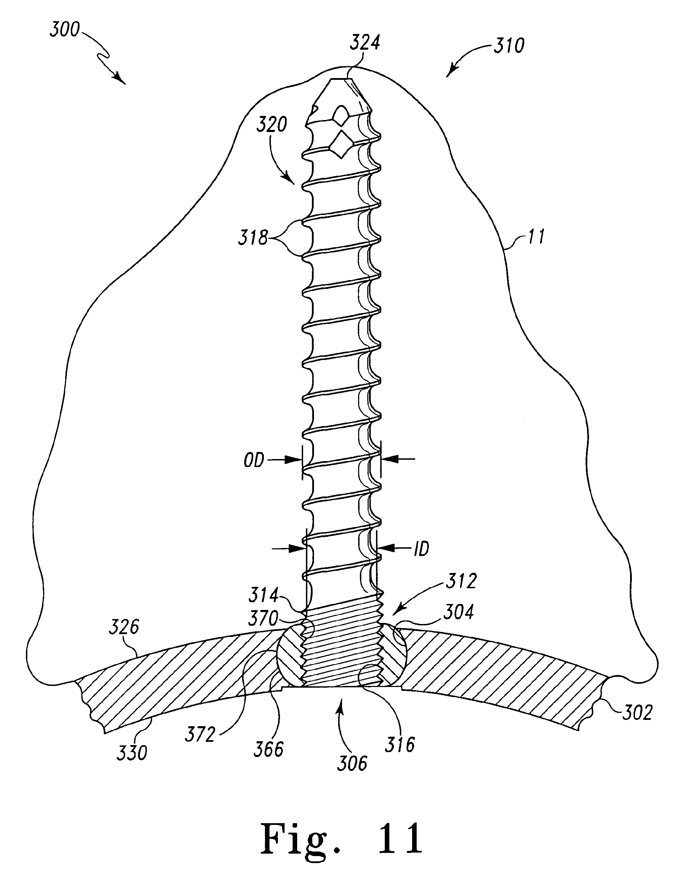

Referring now to FIG. 11, the fastening portion of the hip cup assembly 300 is shown in greater detail. The fastener 310, as shown in FIG. 11, is in the form of a cancellous screw. The fastener 310 includes fastening portion 320 which has an outside diameter OD which may be less than the inside diameter ID of the internal wall or surface 370 of the bushing 366. The fastener 310 further includes the bone threads 318 which define the outside diameter OD. The fastener 310 also includes external threads 314 located on the connecting portion 312 of the fastener 310. As shown in FIG. 11, the external threads 314 are tapered. The external threads 314 of fastener 310 matably engage with the internal threads 316 on the bushing 366.

The bushing 366 is pivotably engageable with the hip cup 302. The bushing 366 includes a radial exterior surface 372 that has a generally spherical shape and is matably fittable with the interior wall or surface 304 of the cup hole or opening 306. The inside diameter ID of the internal threads 316 of the bushing 366 may be larger than the outside diameter OD of the cancellous bone threads 318 on the fastening portion 320 of the fastener 310 to permit the fastening portion 320 of the fastener 310 to slidably pass through the opening 306.

It should be appreciated that, alternatively, the bone threads 318 may have the same pitch as the internal threads 316 of the bushing 366. In this case the fastening portion 320 of the fastener 310 may be threaded through the bushing 366.

It should be appreciated that the external threads 314 and internal threads 316 may be multiple lead threads and may, as shown in FIG. 11, have the same pitch as the single lead threads of the bone threads 318. The use of multiple lead threads will be discussed later in greater detail.

The fastening portion 320 of the fastener 310 may include the tip 324. The tip 324 may optionally include a self-drilling and/or a self-tapping feature to assist in the installing of the fastener 310 to the acetabulum 11.

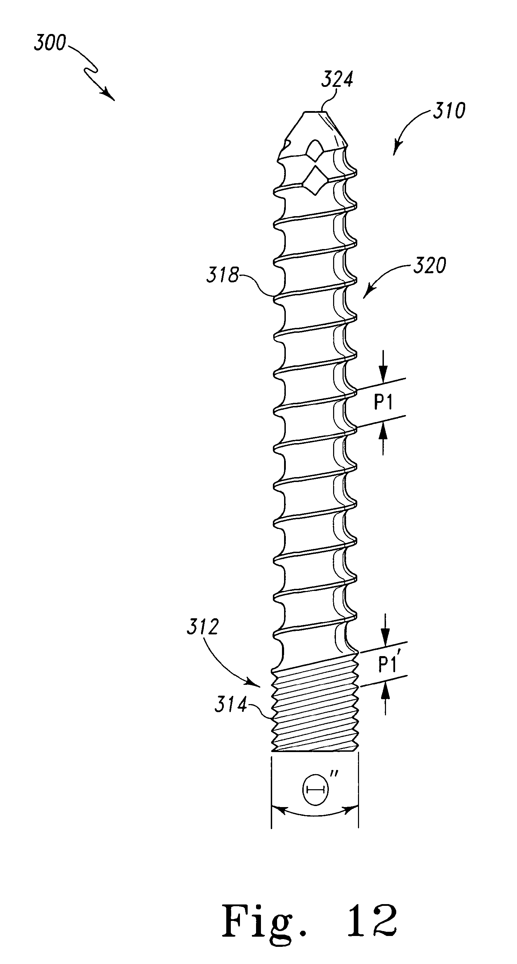

Referring now to FIG. 12, the fastener or screw 310 of the hip cup assembly 300 is shown in greater detail. The fastener 310 includes the fastening portion 320 as well as the connecting portion 312. The connecting portion 312 includes external threads 314 which are tapered and defined by a taper angle θ".

The tapered threads 314 are, as shown in FIG. 12, of a multiple lead variety. For example, as shown in FIG. 12, the threads 314 have a triple lead, meaning that a pitch is equal to three adjacent threads as shown as P1'. In order that the fastener 310 may be installed through the opening 306 in the cup 302 (see FIG. 11), the bone threads 318 in the fastening portion 320 have a pitch P1, which is identical to the pitch P1' of the connecting portion 312.

The bone threads 318 of the fastening portion 320 of the fastener 310 as shown in FIG. 12, are cancellous threads. It should be appreciated that cancellous threads may have a large pitch and may have a substantial thread height to be able to anchor in soft cancellous bone. It should be appreciated that the bone threads 318 may cortical bone. If engaging with cortical bone, the bone threads will have a smaller pitch more suitable for securing in more dense and stronger bone.

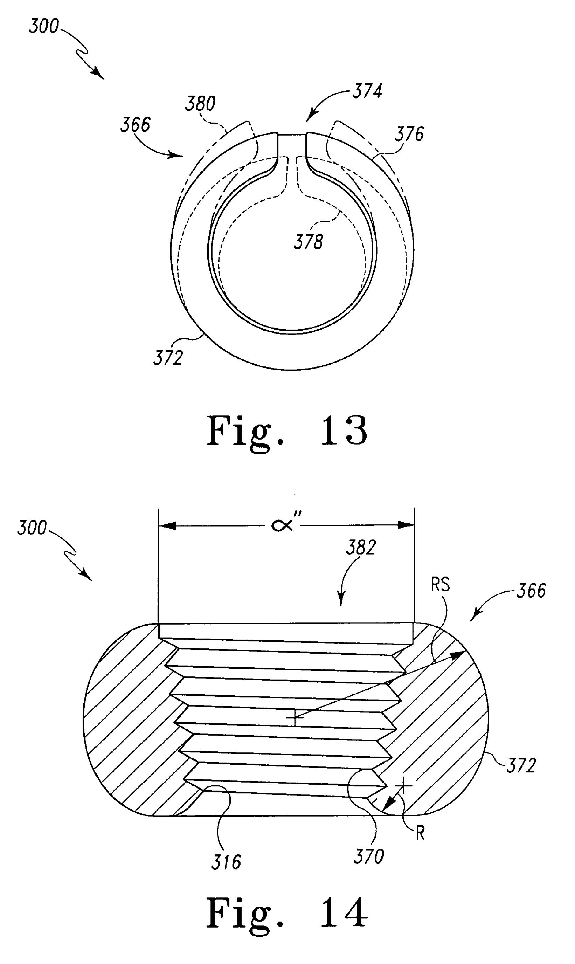

Referring now to FIGS. 13 and 14, the bushing 366 of the cup assembly 300 is shown in greater detail. The bushing 366 is pliable or collapsible so that it may rigidly secure the fastener to the cup. Therefore, the bushing 366 may also be considered as a collet. The bushing or collet 366 is manufactured from any suitable durable material that is compatible with the human body. For example, the collet may be made of a metal, such as a cobalt chromium alloy, a stainless alloy, or a titanium alloy. For example, the bushing 366 may be manufactured from a wrought titanium alloy. Such a wrought titanium alloy is ASTMF-136ELI.

The bushing 366 preferably includes a radial opening or passage way 374 on the periphery of the bushing 366. The passageway 374 extends from the radially exterior surface 372. The bushing 366, as shown in FIG. 13, has a first relaxed position 376 as shown in solid, which represents the shape of the bushing 366 when not assembled into the hip cup 302. The bushing 366 further has a contracted position 378, which represents the position necessary to install the bushing 366 into the hip cup 302. Once installed into the hip cup 302, the bushing 366 returns to the uninstalled relaxed position 376.

The bushing 366 further has an expanded position 380 shown in phantom in which the bushing 366 would be installed in the hip cup and the fastener installed and secured against the bushing 366.

It should be appreciated that as the bushing 366 is expanded as it moves from the relaxed position 376 to the expanded position 380, the radially exterior surface 372 of the bushing 366 expands into locked engagement with the internal wall 304 of the cup 302 (see FIG. 11). The enlargement of the bushing 366 causes a tight interference between the bushing 366 and the hip cup 302, thereby securely locking the bushing 366 in its polyaxially oriented position with minimal stress.

Referring now to FIG. 14, a cross section of the bushing 366 is shown. As shown in FIG. 14, the bushing 366 has a spherical radius RS which defines the radial exterior surface 372 of the bushing 366. By providing a spherical radius RS, the bushing 366 may be oriented into a number of angular positions with respect to the hip cup 302.

The internal thread 316 of the bushing 366 has a taper defined by included angle α". The angle α" may be, for example, from 3 to 30°. As shown in FIG. 14, the truncated spherical shape of the radial exterior surface 372 may be modified by corner radius R. It should be appreciated that the internal threads 316 and radial interior surface 370 of the bushing 366 define a central axial opening 382 for receiving the fastener 310.

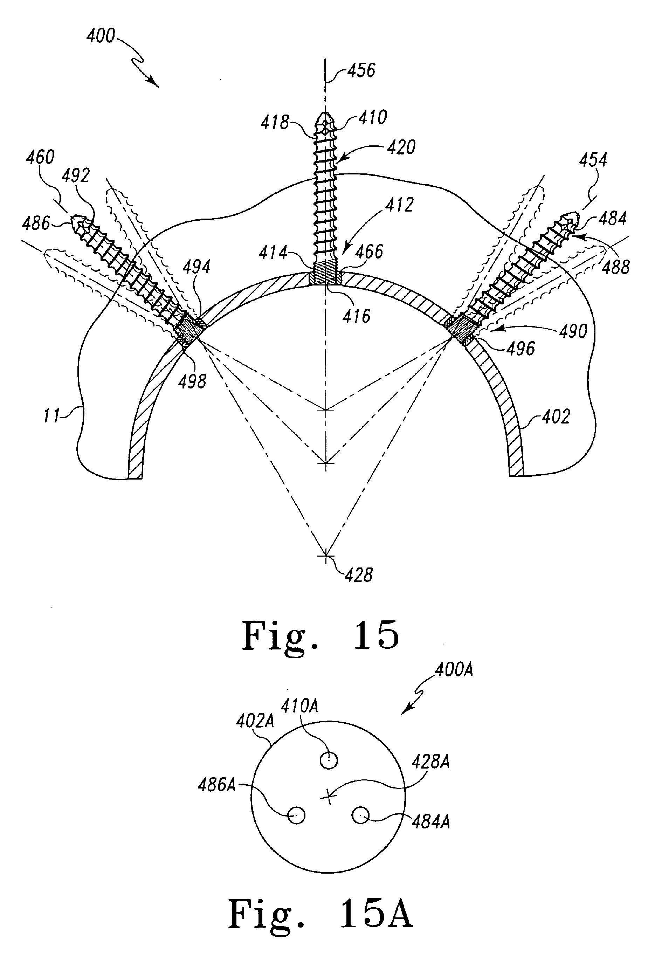

Referring now to FIG. 15, yet another embodiment of the present invention is shown as hip cup assembly 400. The hip cup assembly 400 of FIG. 15 is similar to the hip cup assembly 300 of FIG. 8, except that the hip cup assembly 400 includes a plurality of fasteners.

For example and as shown in FIG. 15, the hip cup assembly 400 includes cup 402 similar to cup 302 of FIG. 8. The hip cup assembly 400 of FIG. 15 also includes a first fastener 410 similar to fastener 310 of FIG. 8. The first fastener 410 includes a connecting portion 412 as well as fastening portion 420.

The connecting portion may, as shown in FIG. 15, include external threads 414. The external threads 414 mate with internal threads 416 formed on bushing 466. Fastening portion 420 includes bone threads 418, which mate with acetabulum 11.

The hip cup assembly 400 of FIG. 15 also includes second fastener 484 as well as third fastener 486. The fasteners 484 and 486 are similar to the first fastener 410. The second fastener 484 includes a fastening portion 488 and a connecting portion 490. Similarly, the third fastener 486 includes a fastening portion 492 and a connecting portion 494. Second fastener 484 cooperates with second bushing 496 and the third fastener 486 cooperates with third bushing 498. The second bushing 496 and the third bushing 498 are similar to the first bushing 466.

The second fastener 484 like the first fastener 410 may be positioned in a plurality of positions. For example and as is shown in FIG. 15, the second fastener 484 may be in the position as shown in solid as well as in the end positions as shown in phantom. Similarly, the third fastener 486 may be positioned in a plurality of locked positions. For example, the third fastener 486 may be in the first position shown in solid as well in second and third positions as shown in phantom. It should be appreciated that the first fastener 410, the second fastener 484, and the third fastener 486 may be positioned in a multitude of positions to accomplish locations that are compatible with portions of the acetabulum more suitable for securement of the hip cup assembly 400 to the acetabulum 11.

As shown in FIG. 15, longitudinal centerline 456 of first fastener 410, longitudinal centerline 454 of the second fastener 484, as well as longitudinal centerline 460 of the third fastener 486 may converge to origin 428 as shown in FIG. 15. It should be appreciated that the orientation of the centerlines 454, 456 and 460 may converge to other positions as shown in phantom in FIG. 15. It should also be appreciated that the longitudinal centerlines of the fasteners 410, 484 and 486 may be skewed with respect to each other in order that the fasteners align with portions of the acetabulum where the best securement can be obtained.

While the hip cup assembly 400 in FIG. 15 shows three fasteners, which are coplanar, it should be appreciated that the fasteners of the hip cup assembly 400 may be spaced more uniformly about the hip cup. For example and as shown in FIG. 15A, hip cup assembly 400A of the present invention may, as shown, include hip cup 402A. The hip cup assembly 400A may include a first fastener 410A, a second fastener 484A and a third fastener 486A that are spaced equally about origin 428A to better support the hip cup 402A.

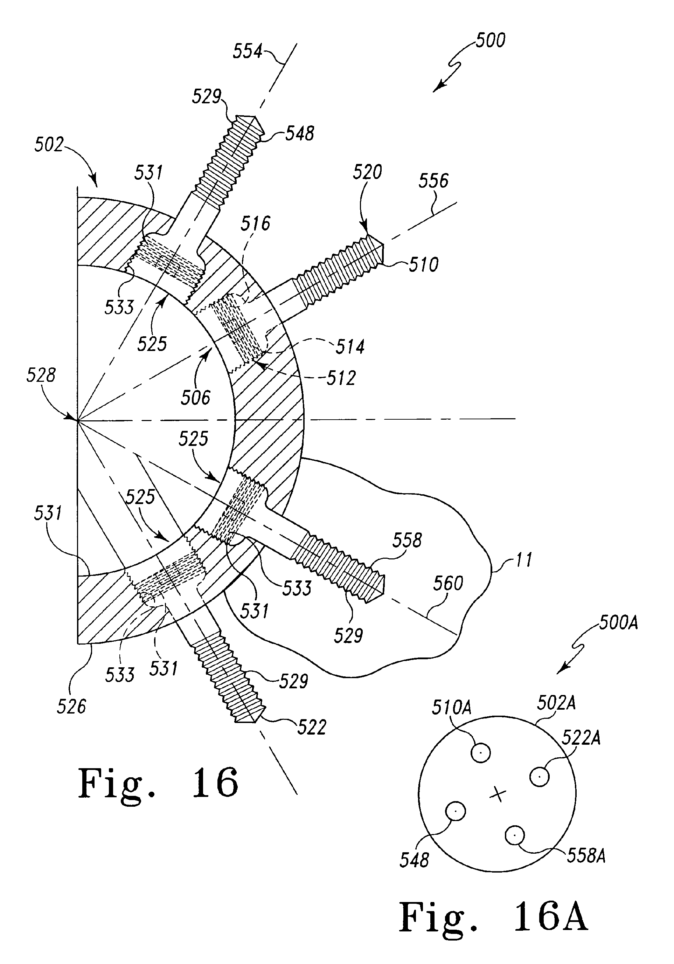

Referring now to FIG. 16, yet another embodiment of the present invention is shown as orthopaedic joint assembly 500. The orthopaedic joint assembly 500 is in the form of a hip cup assembly 500. The hip cup assembly 500 includes a cup 502. The hip cup 502 is similar to the cup 100 of FIG. 4, except that the hip cup assembly 500 includes a plurality of fasteners. Therefore, the hip cup assembly 500 includes fastener 510 similar to first fastener 110 of FIG. 4, as well as additional fasteners, for example, second fastener 548.

While the orthopaedic joint assembly 500 may include only two fasteners, for example, first fastener 510 and second fastener 548, as shown in FIG. 16, the hip cup assembly 500 may also include a third fastener 558, as well as a fourth fastener 522. The fasteners 510, 548, 558 and 522 may, as shown in FIG. 16, be substantially similar.

The first fastener 510 includes a first portion 520 for engagement with acetabulum 11, as well as second or connecting portion 512 for cooperation with the cup 502. The connecting portion 512 may include external threads 514 for cooperation with internal threads 516 formed on opening 506 formed in the cup 502.

Similarly, the second fastener 548, the third fastener 558, and the fourth fastener 522 may include bone threads 529 for cooperation with the acetabulum 11. The second fastener 548, third fastener 558 and fourth fastener 522 may, as shown in FIG. 16, be rigidly secured to the cup 502 by external threads 531 formed on the fasteners which cooperate with internal threads 533 formed in the openings 525 of the cup 502.

As shown in FIG. 16, the first fastener 510 includes a first fastener centerline 556, while the second fastener 548 includes a second fastener longitudinal centerline 554 and the third fastener 558 includes a third fastener centerline 560, etc. The centerlines 554, 556 and 560 as shown in FIG. 16 merge at origin 528. It should be appreciated that the positioning of the centerlines may be elsewhere. The positioning of the centerlines as shown in FIG. 16 may be selected to reach the widest selection of securing locations in the acetabulum 11.

While as shown in FIG. 16, the fasteners may all be coplanar, it should be appreciated that a hip cup assembly may be provided in which the fasteners are more evenly distributed about the hip cup. For example

and as shown in FIG. 16A, a hip cup assembly 500A according to the present invention is shown. The hip cup assembly 500A includes a hip cup 502A which has openings to receive a first fastener 510A, a second fastener 548A, a third fastener 558A and a fourth fastener 522A. The fasteners 510A, 548A, 558A and 522A, as shown in FIG. 16A, may be evenly distributed about the hip cup 502A.

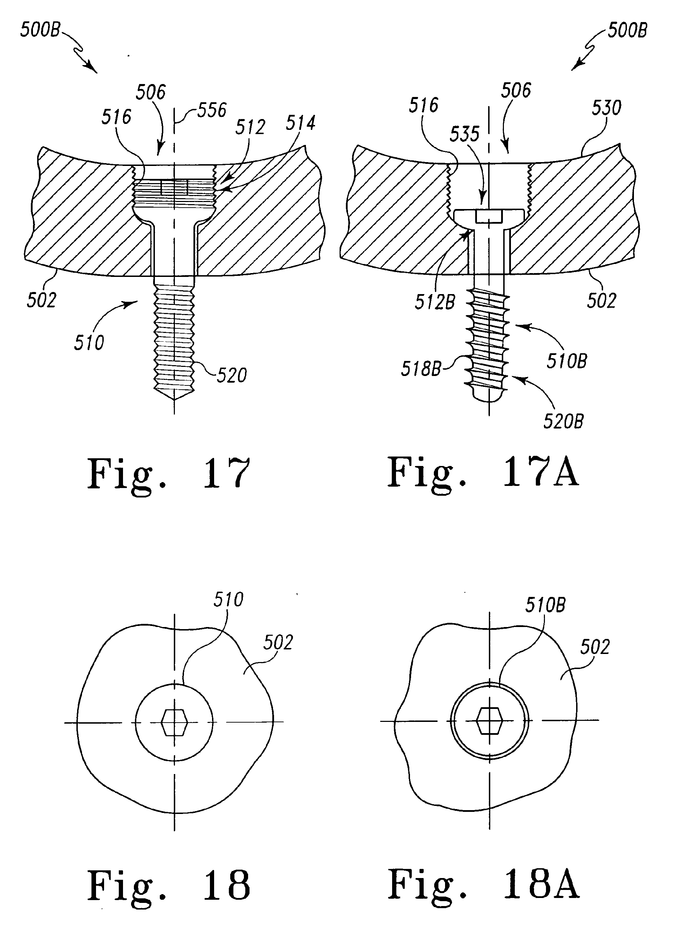

Referring now to FIGS. 17, 17A, 18 and 18A, yet another embodiment of the present invention is shown as hip cup assembly 500B. Hip cup assembly 500B utilizes the hip cup 502 of FIG. 16, but utilizes alternatively the fastener 510 of FIG. 16 and a different fastener. The fastener 510 is rigidly secured to the hip cup 502B. It should be appreciated that the hip cup assembly 500B may, according to the present invention, include both fasteners 510B, which are nonlocking fasteners, as well as the locking fasteners 510 as shown in FIGS. 17 and 18. The hip cup assembly 500B includes hip cup 502 as well as the fastener 510B or the fastener 510.

As shown in FIGS. 17A and 18A, the hip cup assembly 500B alternatively includes fastener 510B having a fastening portion 520B as well as a connecting portion 512B in the form of a head. The head 512B rests on counter bore 535 formed in the opening 506 of the hip cup 502B The fastening portion 520B includes threads, for example, bone threads 518B, which may, as shown in FIG. 17A, be in the form of cancellous threads.

While it should be appreciated that the prosthetic assembly of the present invention may include fasteners which extend outwardly from, as shown in FIGS. 16 and 16A, a origin or center part of the cup. It should be appreciated that for hip cup assemblies with locking fasteners that have a single locking position, different orientations of the fasteners with respect to the cup may be designed for particular conditions of the patient.

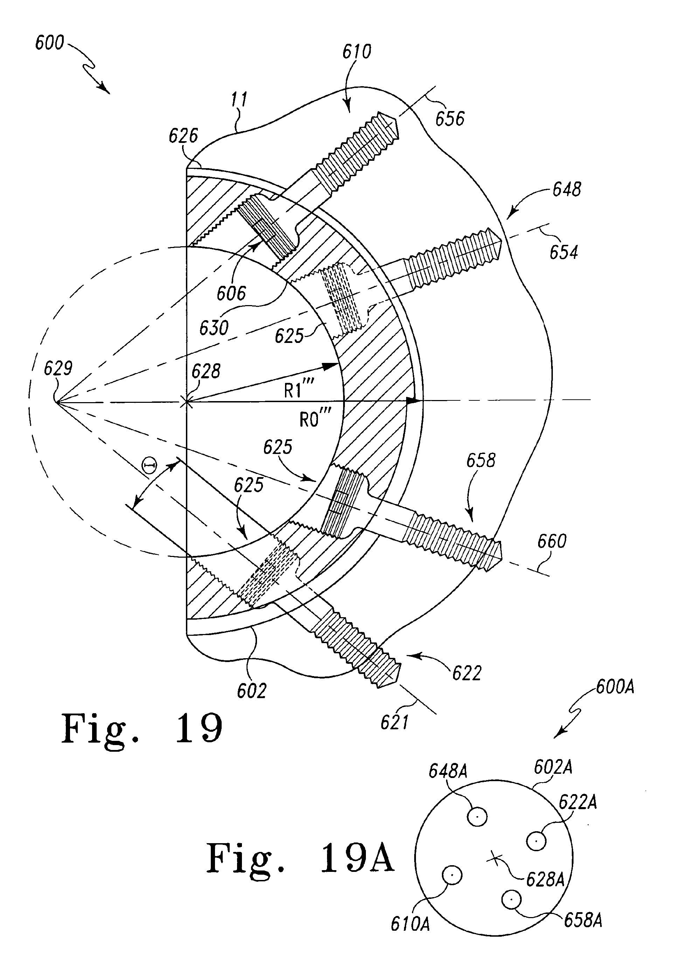

For example and as shown in FIG. 19, the fasteners may be positioned such that they extend more radially inward toward the patient. For example as is shown in FIG. 19, hip cup assembly 600 includes hip cup 602. The hip cup 602 is similar to the hip cup 502 of FIGS. 16, 17 and 17A except that the hip cup 602 includes first opening 606 and additional openings 625, which have centerlines that do not extend to the origin 628 of the cup 602.

For example and as shown in FIG. 19, the hip cup 602 includes an inner periphery 630 defined by radius R1''' which extends outwardly from origin 628. The hip cup 602 is further defined by radius R0''' extending outwardly from origin 628. The outer periphery 626 of the hip cup 602 further includes the first opening 606 as well as additional radial openings 625.

As shown in FIG. 19, the first opening 606 defines a first opening axis centerline 656. The additional radial openings 625 define second openings centerline 654, third opening centerline 660 and fourth opening centerline 621. The opening centerlines 656, 654, 660 and 621 all merge toward origin 629, which is spaced from the origin 628 defining the inner and outer peripheries of the hip cup 602. First fastener 610 is lockably engaged in the hip cup 602 at first opening 606 while second fastener 648, third fastener 658 and fourth fastener 622 are lockably secured in the other openings 625.

While the hip cup assembly 600 in FIG. 19 shows four fasteners, which are coplanar, it should be appreciated that the fasteners of the hip cup assembly 600 may be spaced more uniformly about the hip cup. For example and as shown in FIG. 19A, hip cup assembly 600A of the present invention may, as shown, include hip cup 602A. The hip cup assembly 600A may include a first fastener 610A, a second fastener 648A, a third fastener 658A and a fourth fastener 622A that are spaced equally about origin 628A to better support the hip cup 602A.

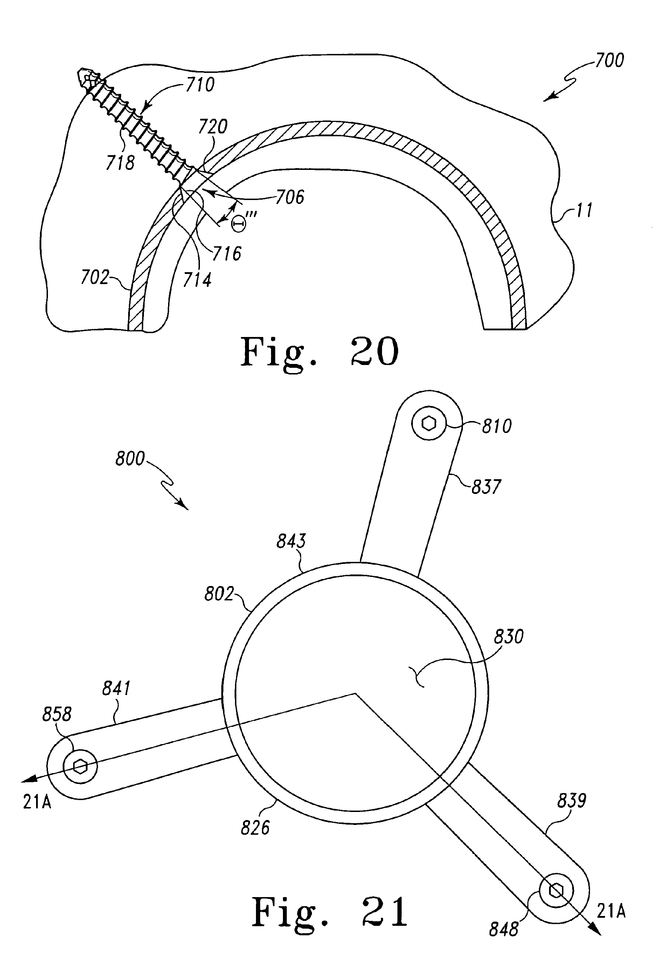

Referring now to FIG. 20, yet another embodiment of the present invention is shown as hip cup assembly 700. The hip cup assembly 700 is similar to the hip cup assembly 100 of FIG. 4 except that fastener 710 includes a connection portion 720, which lockably secures the fastener 710 to hip cup 70 in yet another manner. The fastener 710 further includes a fastening portion 718 for engagement with the acetabulum 11.

The connection portion 720 is different than the connection portion 120 of FIG. 4 in that the connection portion 720 does not include threads. Alternatively, the connection portion 720 utilizes a locking taper to lock the fastener 710 to the hip cup 702. The connection portion 720 includes an external taper 714, which mates with internal taper 716 formed in opening 706 of the hip cup 702. The external taper 714 and internal taper 716 have an included angle θ''' having a small enough angle to provide for self-locking.

For a taper to be self-locking, it must satisfy the following relationship:

$$Tan\frac{\theta'''}{2} < \mu$$

where θ''' is the included taper angle and μ is the coefficient of friction of materials used For example, the angle θ''' may be from about 1 to about 20°.

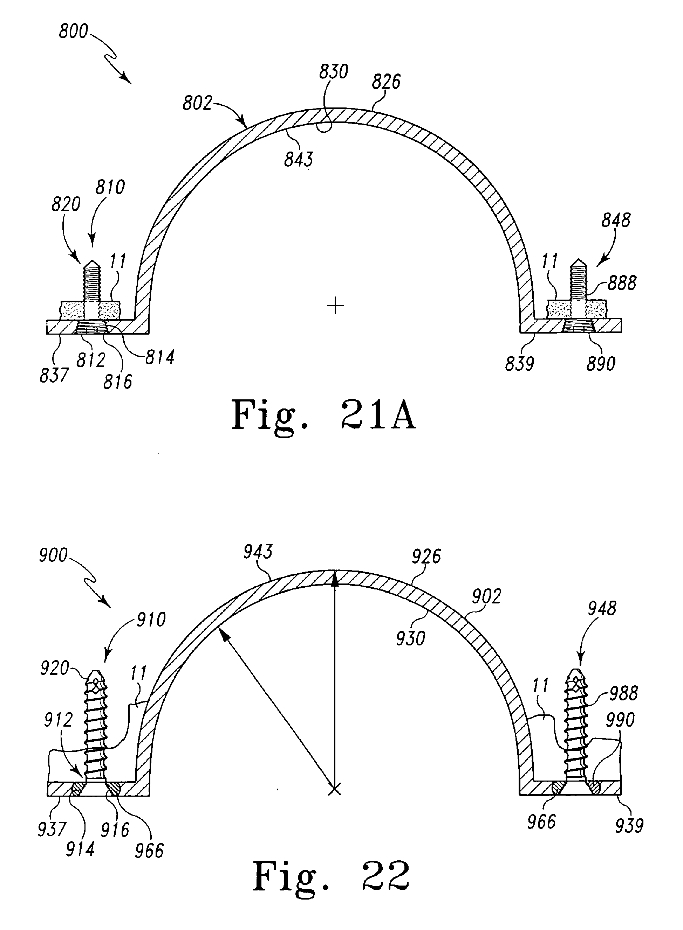

Referring now to FIGS. 21 and 21A, yet another embodiment of the present invention is shown as hip cup assembly 800. Hip cup assembly 800 is similar to hip cup assembly 100 of FIG. 4 except that the hip cup assembly 800 includes a hip cup 802 which includes flanges and a fastener which is connected to the hip cup 802 at the flanges. The hip cup assembly 800 includes a body 843, which has a convex hemispherical outer periphery 826 and a concave inner periphery 830.

Extending from the body 843, are a plurality of flanges. For example and as shown in FIG. 21, a first flange 837 extends outwardly from the body 843. Also a second flange 839 extends outwardly from the body 843 and a third flange 841 also extends outwardly from the body 843. As shown in FIG. 21, the flanges 837, 839 and 841 extend in diverging directions.

A first fastener 810 is fixably secured to the first flange 837. Similarly, a second fastener 848 is fixably secured to the second flange 839. A third fastener 858 is fixably secured to the third flange 841.

Referring now to FIG. 21A, the first fastener 810 and the second fastener 848 are shown in greater detail. The first fastener 810 includes a connection portion 812 for connecting with the first flange 837 of the hip cup 802. The first fastener 810 also includes a fastening portion 820 having bone threads thereon for connection with the acetabulum 11. The connection portion 812 may, as shown in FIG. 21A, include external threads 814 for cooperation with internal threads 816 on the first flange 837 of the hip cup 802. It should be appreciated that the external threads 814 and the internal threads 816 may be tapered to assure a rigid connection.

The hip cup assembly 800 further includes the second fastener 848, which includes a fastening portion 888 and a connection portion 890. The fastening portion 888 engages with the acetabulum 11 and the connecting portion 890 engages with second flange 839 of the cup 802.

Referring now to FIG. 22, yet another embodiment of the present invention is shown as hip cup assembly 900. Hip cup assembly 900 is similar to the hip cup assembly 800 of FIGS. 21 and 21 A, except that the hip cup assembly 900 further includes bushings positioned between the fasteners and the flanges of the hip cup assembly. For example and as shown in FIG. 22, the hip cup assembly 900 includes a hip cup 902 including a hemispherical body 943 and spaced apart flanges 937 and 939. The body 943 includes a hemispherical concave inner periphery 930 and a spaced apart convex outer periphery 926. The first flange 937 extends outwardly from the body 943 and the second flange 939 also extends outwardly from the body 943 in a direction opposed to the first flange 937.

The hip cup assembly 900 further includes first fastener 910. The first fastener 910 is fixably secured to first bushing 966. The first fastener 910 includes a fastening portion 920 for cooperation with the acetabulum 11 and a connection portion 912. The connection portion 912 includes external tapered threads 914, which mate with internal threads 916 formed on bushing 966. The bushing 966 permits pivotable polyaxial lockable motion of the fastener 910 with respect to the cup 902.

The hip cup assembly 900 further includes a second fastener 948, which is similar to the first fastener 910. The second fastener 948 includes a fastening portion 988 for cooperation with the acetabulum 11 as well as a connection portion 990, which lockably cooperates with bushing 996 to lock the second fastener 948 to the flange 939 of the hip cup 902 in a chosen one of multiple polyaxial locked positions.

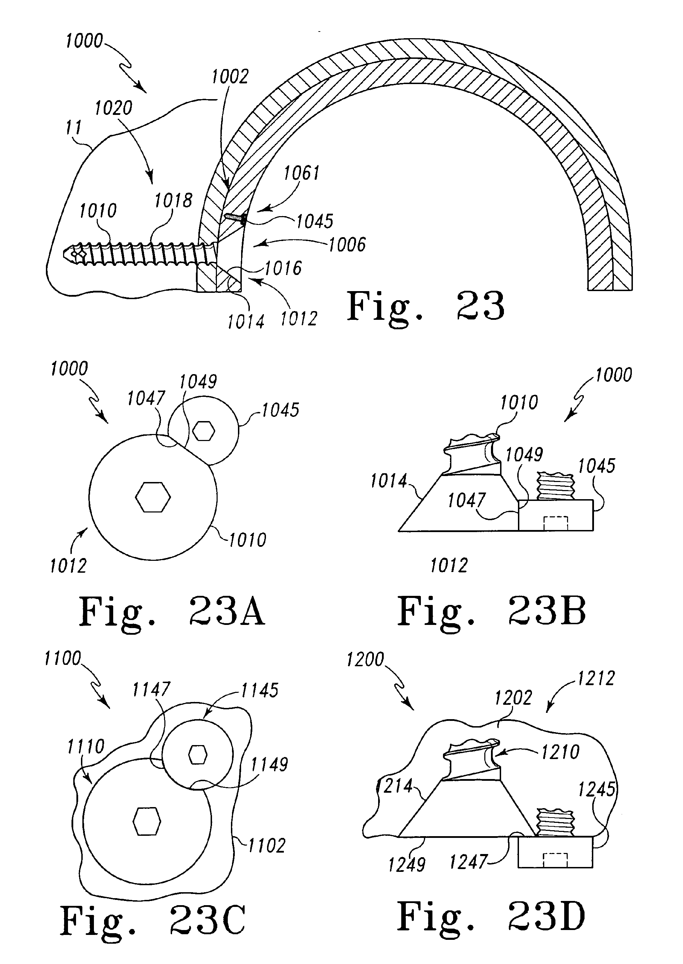

Referring now to FIG. 23, yet another embodiment of the present invention is shown as hip cup assembly 1000. Hip cup assembly 1000 includes hip cup 1002, which is similar to the hip cup 102 of FIG. 4, except that the hip cup 1002 includes a locking screw opening 1006 to assist in locking fastener 1010 to cup 1002. The hip cup assembly 1000 includes in addition to hip cup 1002, the first fastener 1010 and a locking screw 1045. The locking screw 1045 is threadably secured to hip cup 1002 at the first locking screw opening 1061.

The hip cup assembly 1002 includes the first fastener 1010, which is lockably and fixably secured to the hip cup 1002 at opening 1006. The first fastener 1010 includes a fastening portion 1020 for securement to the acetabulum 11, as well as a connecting portion 1012. The connecting portion 1012 may include a threadable locking engagement or, as is shown in FIG. 23, a self-locking taper connection.

As shown in FIG. 23, the connecting portion 1012 includes an external taper 1014 formed on the connecting portion 1012 of the first fastener 1010. The external taper 1014 locks into internal taper 1016 formed in opening 1006 of the hip cup 1002. The fastening portion 1020 includes bone threads 1018 for securement to the acetabulum 11.

Referring now to FIGS. 23A and 23B, the connecting of the locking screw 1045 to the fastener 1010 is shown in greater detail. The locking screw 1045 includes a locking screw flat 1047, which fits against fastening flat 1049 formed on the fastener 1010.

Referring now to FIG. 23C, yet another embodiment of the present invention is shown as hip cup assembly 1100. The hip cup assembly 1100 is similar to the hip cup assembly 1000 of FIGS. 23, 23A and 23B, except that the locking screw and fastener cooperate in a somewhat different fashion. As shown in FIG. 23C, the locking screw 1145 includes an external periphery 1147, which mates with scallop 1149 formed in fastener 1110. The locking screw 1145 and the fastener 1110 cooperate with cup 1102 to form hip cup assembly 1100.

Referring now to FIG. 23D, yet another embodiment of the present invention is shown as hip cup assembly 1200. The hip cup assembly 1200 includes a hip cup 1202 similar to the hip cup 1002 of FIGS. 23, 23A and 23B. Fastener 1210 and locking screw 1245 of the hip cup assembly 1200 are somewhat different than the locking screw 1045 and the fastener 1010 of the hip cup assembly 1000. The fastener 1210 includes an external taper 1214 that is lockably engaged with the cup 1202. The hip cup assembly 1200 further includes a connecting portion 1212 including locking screw 1245, which defines a recessed face 1247 that mates with top 1249 of the fastener 1210 to secure the fastener 1210 to the hip cup 1202.

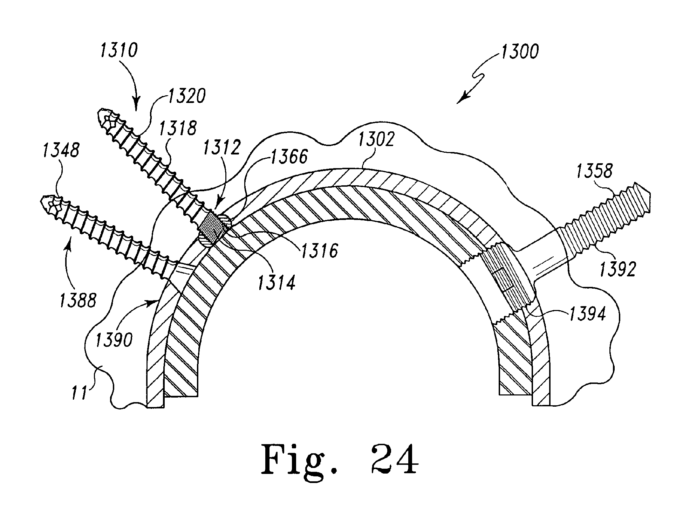

Referring now to FIG. 24, yet another embodiment of the present invention shown as hip cup assembly 1300. The hip cup assembly 1300 includes a hip cup 1302 similar to the hip cup 102 of FIG. 4, except that the hip cup 1302 includes a plurality of fasteners of different types. For example and as shown in FIG. 24, the hip cup assembly 1300 includes a first fastener 1310 including a fastening portion 1320, which comprises cancellous bone threads 1318.

The first fastener 1310 further includes a connecting portion 1312, which includes a bushing 1366 having internal threads 1316 which mate with external threads 1314 located on the connection portion 1312 of the first fastener 1310.

The hip cup assembly 1300 further includes a second fastener 1348 including a fastening portion 1388 for cooperation with acetabulum 11. The fastening portion 1388 may include cancellous threads. The second fastener 1348 further includes a connecting portion 1390 in the form of a locking taper.

The hip cup 1302 further includes a third fastener 1358 having a fastening portion 1392 in the form of, for example, cortical threads and a connection portion 1394 in the form of cylindrical external threads, which mate with cylindrical internal threads on the hip cup 1302.

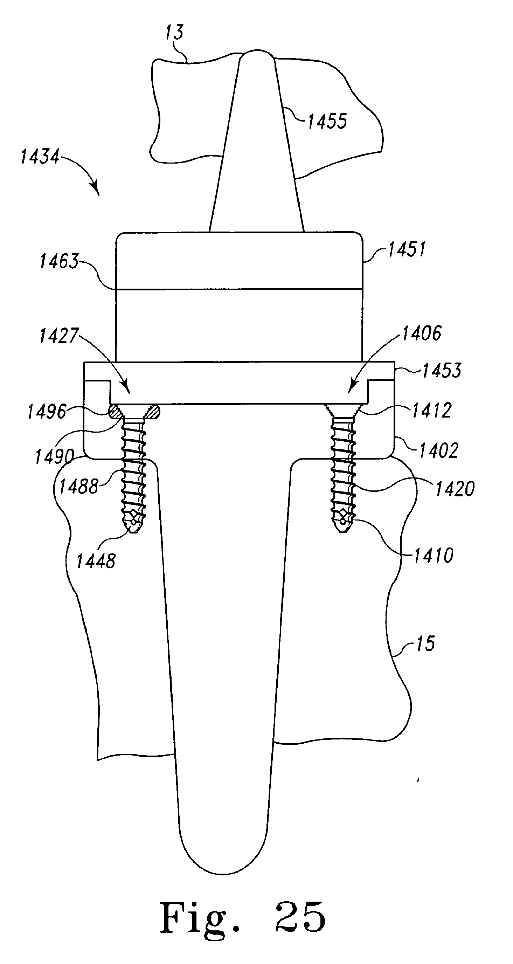

Referring now to FIG. 25, yet another embodiment of the present invention is shown as orthopaedic joint assembly 1434. The orthopaedic joint assembly 1434 includes a femoral component 1451, including a stem 1455 for cooperation with femur 13 and an articulating portion 1463 extending from the stem 1455. The articulating portion 1463 cooperates with bearing 1453.

The bearing 1453 cooperates with tibial component or tibial tray 1402. The tibial tray 1402 includes a first opening 1406 and a spaced apart second opening 1427. The first opening 1406 lockably cooperates with first fastener 1410 while second opening 1427 lockably cooperates with second fastener 1448. The first fastener 1410 includes a fastening portion 1420 for cooperation with tibia 15 and a connecting portion 1412 for lockable connection with the tibial tray 1402.

The second fastener 1448 includes a fastening portion 1488 for cooperation with the tibia 15 and a connection portion 1490 for lockable cooperation with bushing 1496. The bushing 1496 lockably cooperates with the tibial tray 1402 for polyaxial positioning in a lockable fashion with the fastener 1448.

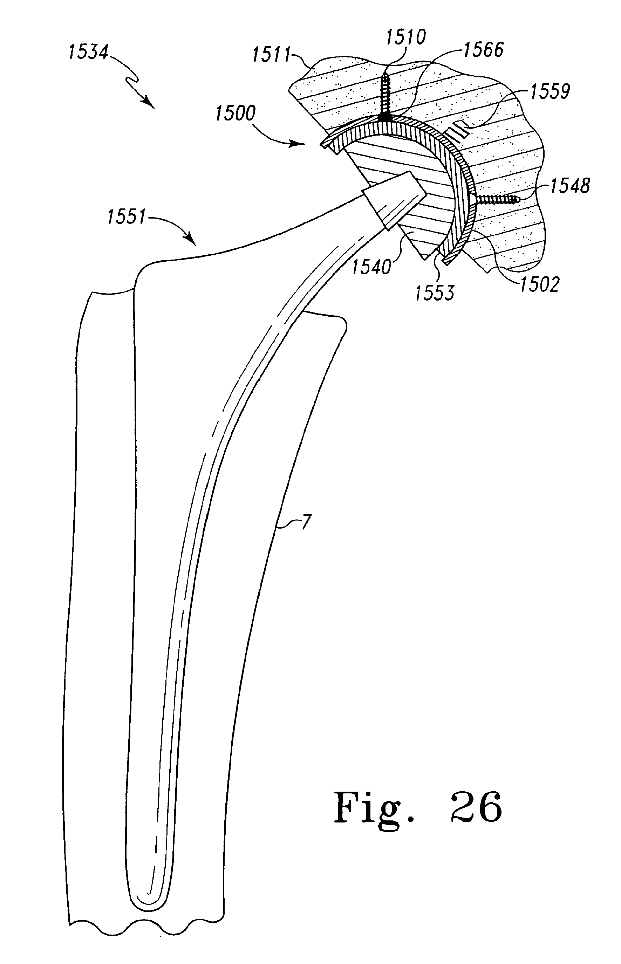

Referring now to FIG. 26, yet another embodiment of the present invention is shown as orthopaedic joint assembly 1534 in the form of a shoulder prosthesis. The shoulder prosthesis 1534 includes a humeral stem 1551 for implantation in humerus 7. The shoulder prosthesis 1534 further includes a head 1540 for positioning on humeral stem 1551. The shoulder prosthesis 1534 further includes a bearing 1553 for pivotal cooperation with the head 1540. The shoulder prosthesis 1534 further includes a glenoid assembly 1500 for implantation to glenoid 1511 and for cooperation with the head 1540. The glenoid assembly 1500 includes glenoid component 1502 as well as first fastener 1510 and second fastener 1548. The glenoid component 1502 may include the bearing 1553 and may be integral with the bearing 1553.

The first fastener 1510 includes a bushing 1566 positioned between the fastener 1510 and the glenoid component 1502. The glenoid assembly 1500 further includes the second fastener 1548, which is lockably secured by a tapered connection to glenoid component 1502. The glenoid component 1502 further includes a peg 1559 for engagement in glenoid 1511.

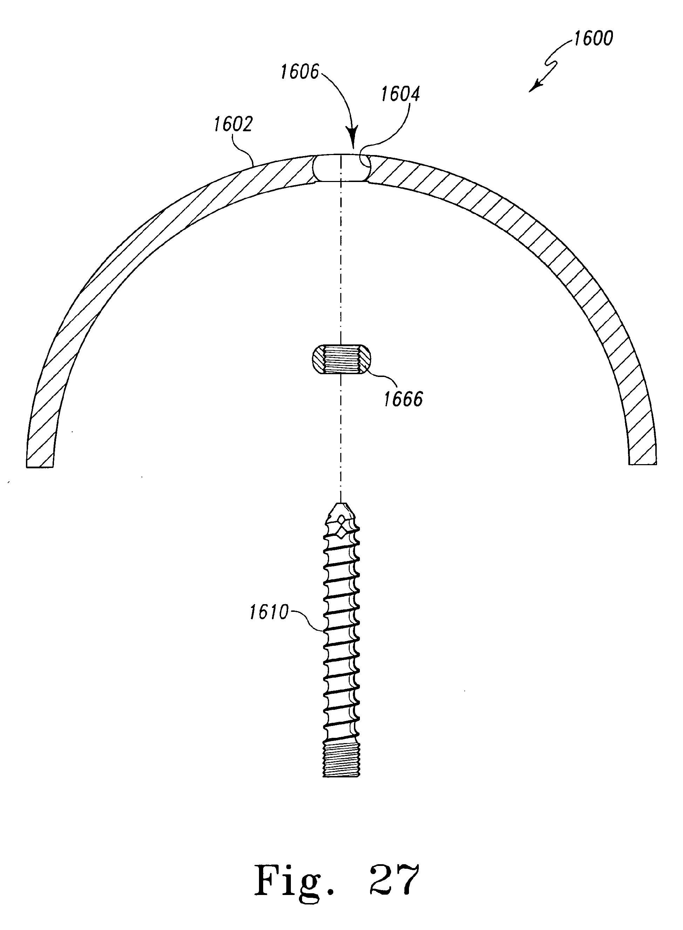

Referring now to FIG. 27, yet another embodiment of the present invention is shown as hip cup kit 1600. The kit 1600 includes cup 1602 having a opening 1606 defined by interior wall 1604. The hip cup kit 1600 further includes a fastener 1610 and a bushing 1666.

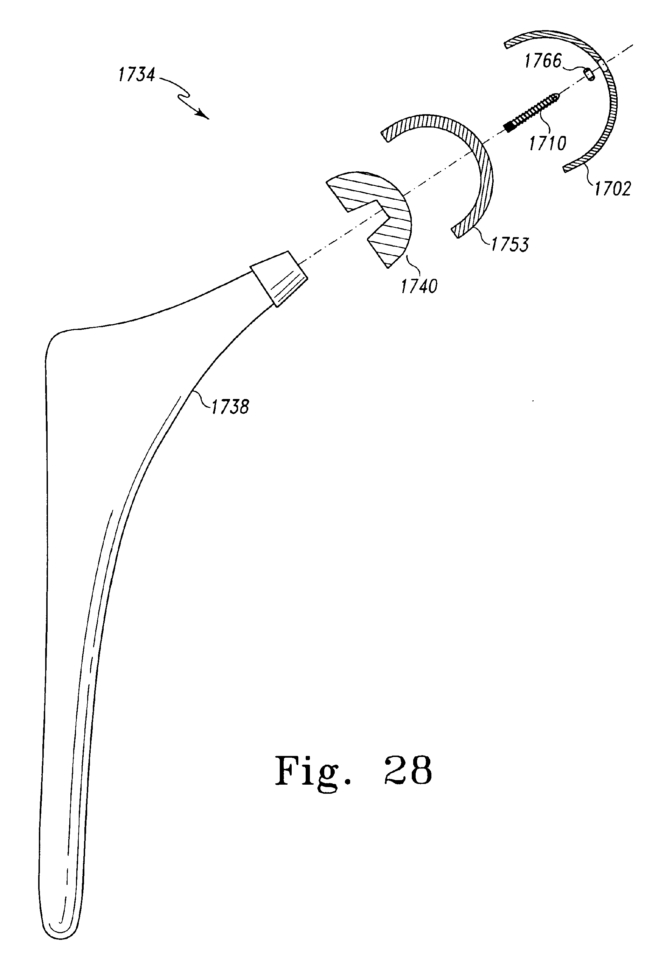

Referring now to FIG. 28, yet another embodiment of the present invention is shown as prosthetic assembly kit 1734. The prosthetic kit 1734 is in the form of a hip prosthesis including a hip stem 1738, a hip head 1740, and a liner or bushing 1753. The hip prosthesis kit 1734 further includes a hip cup 1702, a bushing 1766 and a fastener 1710.

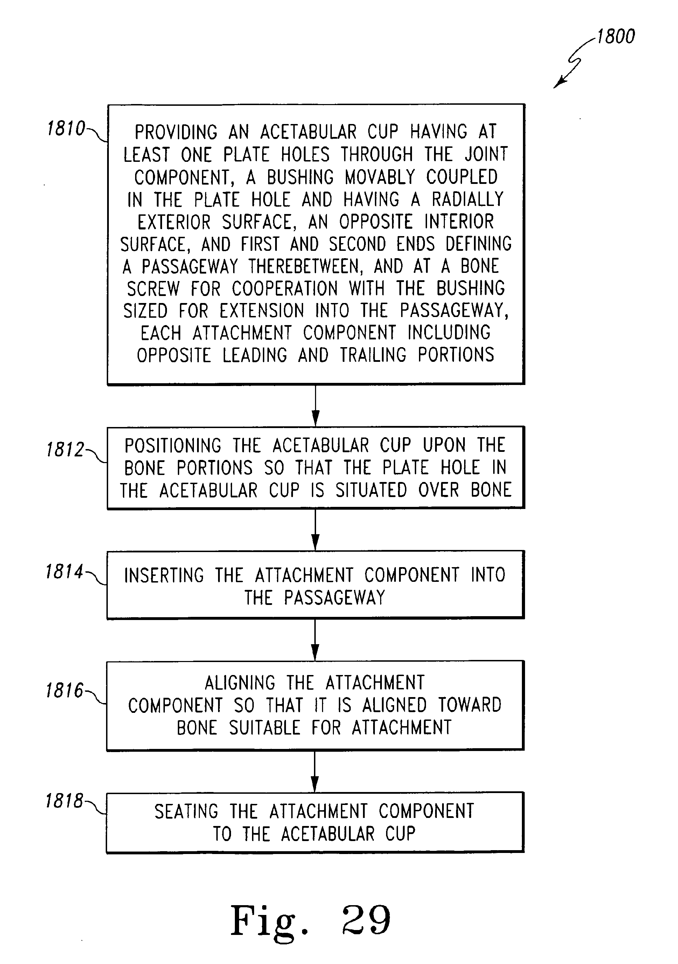

Referring now to FIG. 29, yet another embodiment of the present invention is shown as method 1800 for performing orthopaedic hip arthroplasty on a patient. The method 1800 includes a first step 1810 providing an acetabular cup assembly. The acetabular cup assembly includes a cup having at least one hole through the cup. The cup assembly further includes a bushing movably positionable in the hole of the cup. The bushing includes a radial exterior surface, an opposed interior surface, and first and second ends that define a passageway between the first and second ends. The cup assembly further includes a bone screw for cooperation with the bushing size for extension into the passageway. The attachment component includes an opposed leading and trailing portions.

The method 1800 further includes a second step 1812 of positioning the acetabular cup upon the bone portion so that the opening in the cup is situated over bone. The method 1800 further includes a third step 1814 of inserting the attachment component into the passageway. The method 1800 further includes a fourth step 1816 of aligning the attachment component so that it is aligned toward bone suitable for attachment. The method 1800 further includes a fifth step 1818 of seating the attachment component to the acetabular cup.

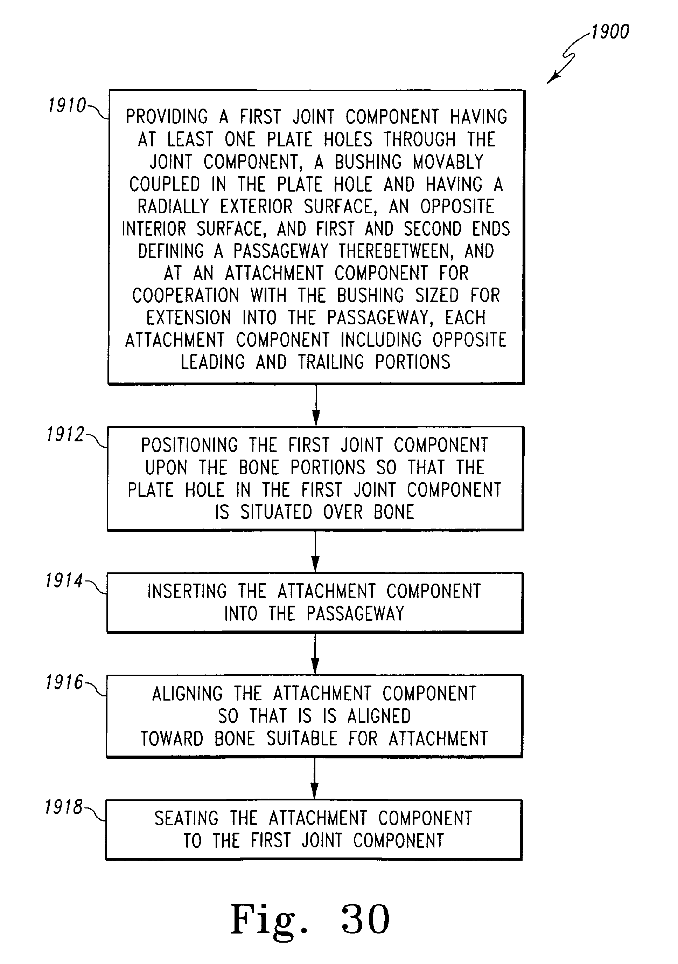

According to the present invention and referring now to FIG. 30, yet another embodiment of the present invention is shown as surgical procedure 1900. The surgical procedure 1900 includes a first step 1910 of providing a first joint component assembly. The first joint component assembly includes a first joint component having a hole through the joint component. The joint component assembly also includes a bushing movably coupled to the component in the hole and having a radial exterior surface. The bushing further includes an opposed interior surface and first and second ends defining a passageway between the first and second ends. The first joint component assembly further includes an attachment component for cooperation with the bushing and sized for extension into the passageway. The attachment component includes opposed leading and trailing portions.

The method 1900 further includes a second step 1912 of positioning the joint component with the bone portions so that the hole in the first joint component is situated over bone. The method 1900 includes a third step 1914 of inserting the attachment component into the passageway. The method 1900 includes a fourth step 1916 of aligning the attachment component so that it is aligned toward bones suitable for attachment. The method 1900 further includes a fifth step 1918 of seating the attachment component to the first joint component.

**Claims**

1.  An acetabular cup assembly for attachment to a acetabulum, which comprises an acetabular cup including a first connector; and a fastener for cooperation with the cup, the fastener including a second connector, the first connector and the second connector cooperating to rigidly connect the fastener to the cup.

2.  The acetabular cup assembly as in claim 1, in which the acetabular cup further includes a third connector; and which includes a second fastener for cooperation with the cup, the second fastener including a fourth connector, the third connector and the fourth connector cooperating to rigidly connect the second fastener to the cup.

3.  The acetabular cup assembly as in claim 2, in which the acetabular cup includes a fifth connector; and which includes a third fastener for cooperation with the cup, the third fastener including a sixth connector, the fifth connector and the sixth connector cooperating to rigidly connect the third fastener to the cup.

4.  The acetabular cup assembly as in claim 3, in which the first fastener, the second fastener and the third fastener define respective longitudinal axes and in which the longitudinal axes are approximately parallel to each other.

5.  The acetabular cup assembly as in claim 2, in which the first fastener and the second fastener have a portions which have a generally uniform cross section for engagement with the acetabulum.

6.  The acetabular cup assembly as in claim 2, in which each of the first fastener and the second fastener includes a portion whose surface is adapted for bone ingrowth.

7.  The acetabular cup assembly as in claim 2, in which the first and third connectors of the cup, the second connector of the first fastener, and the fourth connector of the second fastener include comprise threads.

8.  The acetabular cup assembly as in claim 7, in which the threads are tapered threads.

9.  The acetabular cup assembly as in claim 1, which includes a bushing positioned between the first connector and the second connector of the first fastener, the bushing adapted to position the fastener rigidly in the cup against pivoting.

10. A fastener assembly for use to secure an acetabular cup to an acetabulum, which comprises:

    a fastener having a fastening portion for cooperation with the acetabulum and a connecting portion; and
    a bushing including a generally spherical outer periphery and an interior wall defining central opening extending through it, the interior wall cooperating with the connecting portion of the fastener.

11. The fastener assembly as in claim 10, in which the fastening portion of the fastener has a generally uniform cross section.

12. The fastener assembly as in claim 11, in which the fastening portion of the fastener has a generally circular cross section.

13. A fastener for securing an acetabular cup to an acetabulum comprising, which comprises:

    a fastening portion for cooperation with the acetabulum; and
    a connecting portion for connection with the cup.

Fig. 1

Fig. 1A

200A

210A

212A

202A

214A

216A

206A

## Fig.   1B

200B

210B

212B

202B

214B

216B

206B

## Fig.   1C

200C

202C

210C

248C

254C

## Fig.   1D

## Fig. 2
### (Prior Art)

## Fig. 3
### (Prior Art)

Fig. 4

Fig. 4A

Fig. 4B

Fig. 5

Fig. 6

Fig. 7

Fig. 7A

# Fig. 8

Fig. 9

Fig. 10

Fig. 11

Fig. 12

300

380

366

374

376

378

372

# Fig. 13

300

α″

382

RS

366

372

316

370

R

# Fig. 14

Fig. 15

Fig. 15A

Fig. 16

Fig. 16A

500B

506

516    556    512    514

502

510

520

**Fig. 17**

500B

506

516    535    530

512B    502

518B    510B

520B

**Fig. 17A**

510

502

**Fig. 18**

510B

502

**Fig. 18A**

Fig. 19

Fig. 19A

Fig. 20

Fig. 21

Fig. 21A

Fig. 22

Fig. 23

Fig. 23A

Fig. 23B

Fig. 23C

Fig. 23D

Fig. 24

Fig. 25

Fig. 26

Fig. 27

Fig. 28

_1800_

1810 — PROVIDING AN ACETABULAR CUP HAVING AT LEAST ONE PLATE HOLES THROUGH THE JOINT COMPONENT, A BUSHING MOVABLY COUPLED IN THE PLATE HOLE AND HAVING A RADIALLY EXTERIOR SURFACE, AN OPPOSITE INTERIOR SURFACE, AND FIRST AND SECOND ENDS DEFINING A PASSAGEWAY THEREBETWEEN, AND AT A BONE SCREW FOR COOPERATION WITH THE BUSHING SIZED FOR EXTENSION INTO THE PASSAGEWAY, EACH ATTACHMENT COMPONENT INCLUDING OPPOSITE LEADING AND TRAILING PORTIONS

1812 — POSITIONING THE ACETABULAR CUP UPON THE BONE PORTIONS SO THAT THE PLATE HOLE IN THE ACETABULAR CUP IS SITUATED OVER BONE

1814 — INSERTING THE ATTACHMENT COMPONENT INTO THE PASSAGEWAY

1816 — ALIGNING THE ATTACHMENT COMPONENT SO THAT IT IS ALIGNED TOWARD BONE SUITABLE FOR ATTACHMENT

1818 — SEATING THE ATTACHMENT COMPONENT TO THE ACETABULAR CUP

# Fig. 29

*1900*

1910 — PROVIDING A FIRST JOINT COMPONENT HAVING AT LEAST ONE PLATE HOLES THROUGH THE JOINT COMPONENT, A BUSHING MOVABLY COUPLED IN THE PLATE HOLE AND HAVING A RADIALLY EXTERIOR SURFACE, AN OPPOSITE INTERIOR SURFACE, AND FIRST AND SECOND ENDS DEFINING A PASSAGEWAY THEREBETWEEN, AND AT AN ATTACHMENT COMPONENT FOR COOPERATION WITH THE BUSHING SIZED FOR EXTENSION INTO THE PASSAGEWAY, EACH ATTACHMENT COMPONENT INCLUDING OPPOSITE LEADING AND TRAILING PORTIONS

1912 — POSITIONING THE FIRST JOINT COMPONENT UPON THE BONE PORTIONS SO THAT THE PLATE HOLE IN THE FIRST JOINT COMPONENT IS SITUATED OVER BONE

1914 — INSERTING THE ATTACHMENT COMPONENT INTO THE PASSAGEWAY

1916 — ALIGNING THE ATTACHMENT COMPONENT SO THAT IS IS ALIGNED TOWARD BONE SUITABLE FOR ATTACHMENT

1918 — SEATING THE ATTACHMENT COMPONENT TO THE FIRST JOINT COMPONENT

# Fig. 30